(19) Europäisches Patentamt
European Patent Office
Office européen des brevets

(11) Veröffentlichungsnummer: **0 167 912**
**B1**

(12) # EUROPÄISCHE PATENTSCHRIFT

(45) Veröffentlichungstag der Patentschrift: **03.01.90**

(51) Int. Cl.[5]: **C 09 K 19/14**, C 09 K 19/20, C 09 K 19/34, C 09 K 19/42, C 09 K 19/46, G 02 F 1/133

(21) Anmeldenummer: **85107760.2**

(22) Anmeldetag: **22.06.85**

(54) **Flüssigkristalline Gemische enthaltend Komponenten mit einer 4-Alkenyl- oder 2Z-Alkenyl-Seitenkette.**

(30) Priorität: **12.07.84 CH 3385/84**
**06.05.85 CH 1899/85**

(43) Veröffentlichungstag der Anmeldung: **15.01.86 Patentblatt 86/03**

(45) Bekanntmachung des Hinweises auf die Patenterteilung: **03.01.90 Patentblatt 90/01**

(84) Benannte Vertragsstaaten: **CH DE FR GB IT LI SE**

(56) Entgegenhaltungen:
**EP-A- 0 097 033**
**US-A- 4 364 838**

(73) Patentinhaber: **F. HOFFMANN-LA ROCHE AG**
**Postfach 3255**
**CH-4002 Basel (CH)**

(72) Erfinder: **Petrzilka, Martin, Dr.**
**Schwarzackerstrasse 54**
**CH-4303 Kaiseraugst (CH)**
Erfinder: **Schadt, Martin, Dr.**
**Liestalerstrasse 77**
**CH-4411 Seltisberg (CH)**

(74) Vertreter: **Zimmermann, Hans, Dr. et al**
**Grenzacherstrasse 124 Postfach 3255**
**CH-4002 Basel (CH)**

**EP 0 167 912 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue flüssigkristalline Gemische, welche Komponenten mit einer 4-Alkenyl- oder einer 2Z-Alkenyl-Seitenkette enthalten, sowie die Verwendung dieser Verbindungen und Mischungen für elektro-optische Zwecke. Die Erfindung betrifft ebenfalls die neuen 4-Alkenyl- und 2Z-Alkenylverbindungen und deren Herstellung.

Flüssige Kristalle haben in den letzten Jahren vor allem als Dielektrika in Anzeigevorrichtungen stark an Bedeutung gewonnen, da die optischen Eigenschaften solcher Substanzen durch eine angelegte Spannung beeinflusst werden können. Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Zelle), den Schadt-Helfrich-Effekt (Drehzelle), dem Gast/Wirt-Effekt (Guest/Host-Zelle) oder einem cholesterisch-nematischen Phasenübergang (Phase-Change-Zelle).

Die verwendeten Flüssigkristalle müssen eine gute Stabilität gegenüber Wärme, Feuchtigkeit, Luft, elektromagnetischer Strahlung, elektrischen Feldern und dergleichen besitzen. Ferner sollten sie farblos sein, kurze Ansprechzeiten und niedere Viskosität aufweisen und einen guten Kontrast ergeben. Da Flüssigkristalle üblicherweise als Mischungen zur Anwendung gelangen, ist es zudem wichtig, dass die Komponenten untereinander gut mischbar sind und dabei eine nematische bzw. cholesterische Mesophase ausbilden. Weitere Eigenschaften, wie beispielsweise die elektrische Leitfähigkeit, die Schwellenspannung, die Multiplexierbarkeit und die dielektrische Anisotropie, müssen je nach verwendetem Zellentyp unterschiedliche Bedingungen erfüllen.

US-A-4 364 838 beschreibt ein flüssigkristallines Gemisch aus 2-(4-Cyanophenyl)dioxanen, 2-(4-Cyanophenyl)-pyrimidinen, 4-Cyanophenyl-benzoaten und gegebenenfalls weiteren Zusätzen. EP-A-0 097 033 offenbart Phenethyldioxane und Phenethylpyrimidine als Flüssigkristallkomponenten. Die unpolaren endständigen Restes sind Alkylreste oder in der letzteren Publikation auch Alkoxy-, Alkylcarbonyloxy- oder Alkoxycarbonyloxyreste.

Gegenstand der vorliegenden Erfindung sind flüssigkristalline Gemische enthaltend eine oder mehrere Verbindungen der allgemeinen Formel

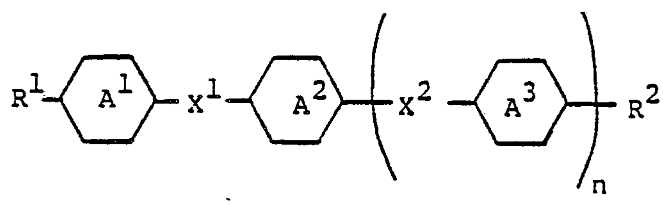 I

worin n für die Zahl 0 oder 1 steht; $X^1$ und $X^2$ einfache Kovalenzbindungen bezeichnen oder eines der Symbole $X^1$ und $X^2$ auch —COO—, —OOC— oder —$CH_2CH_2$— bezeichnet; die Ringe $A^1$, $A^2$ und $A^3$ 1,4-Phenylen, 2-Fluor-1,4-phenylen oder trans-1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $R^1$ 4-Alkenyl oder an einem Cyclohexylring auch 2Z-Alkenyl bedeutet; und $R^2$ Alkyl, Alkoxy, —CN oder —NCS bezeichnet.

Des Ausdruck « 4-Alkenyl » bedeutet im Rahmen der vorliegenden Erfindung Reste wie 4-Pentenyl und die E- und/oder Z-Form von 4-Hexenyl, 4-Heptenyl, 4-Octenyl, 4-Nonenyl, 4-Decenyl, 4-Undecenyl und 4-Dodecenyl. Der Ausdruck « 2Z-Alkenyl » umfasst Reste wie Allyl, 2Z-Butenyl, 2Z-Pentenyl, 2Z-Hexenyl, 2Z-Heptenyl, 2Z-Octenyl, 2Z-Nonenyl, 2Z-Decenyl, 2Z-Undecenyl und 2Z-Dodecenyl. Der Ausdruck « Alkyl » umfasst geradkettige und verzweigtkettige Alkylreste, wie Methyl, Aethyl, Propyl, Isopropyl, Butyl, 2-Methylbutyl, Pentyl, Hexyl, Heptyl, Octyl, Nonyl, Decyl, Undecyl und Dodecyl. Der Ausdruck « Alkoxy » umfasst Alkyloxyreste, worin der Alkylteil die obige Bedeutung hat. Der Ausdruck « Halogen » bedeutet im Rahmen der vorliegenden Erfindung Chlor, Brom oder Jod. Der Ausdruck « Alkalimetall » umfasst Lithium, Natrium und Kalium.

Es wurde gefunden, dass die Verbindungen der Formel I bzw. deren Mischungen die eingangs erwähnten, erforderlichen Eigenschaften aufweisen und zudem niedere Viskositäten, insbesondere eine verbesserte Rotationsviskosität $\gamma_1$ besitzen und vergleichsweise niedrige Schwellenspannungen ermöglichen. Ferner besitzen die Verbindungen der Formel I ein günstiges Verhältnis der elastischen Konstanten $k_{33}$ (bend) und $k_{11}$ (splay) und ergeben somit steile Transmissionskurven und eine gute Multiplexierbarkeit.

Die Verbindungen der Formel I und deren Mischungen können grundsätzlich in beliebigen elektro-optischen Vorrichtungen angewendet werden. Vorzugsweise werden sie jedoch in Drehzellen und in Guest/Host-Zellen verwendet.

Die Herstellung der erfindungsgemässen Mischungen und der elektro-optischen Vorrichtungen kann

in an sich bekannter Weise erfolgen.

Zweckmässigerweise enthalten die erfindungsgemässen Mischungen ein flüssigkristalline Trägermaterial und eine oder mehrere Verbindungen der Formel I. Der Anteil an Verbindungen der Formel I kann in relativ breiten Grenzen variieren. Im allgemeinen enthalten die erfindungsgemässen Mischungen mindestens etwa 1 Gew.-% an Verbindungen der Formel I. Vorzugsweise beträgt der Anteil an Verbindungen der Formel I etwa 5-50 Gew.-% ;

Die erfindungsgemässen Mischungen können neben einer oder mehreren Verbindungen der Formel I übliche Flüssigkristallkomponenten enthalten, wie z. B. Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Terphenyl, Phenylcyclohexane, Cyclohexylbiphenyle, Phenylpyrimidine, Diphenylpyrimidine, Cyclohexylphenylpyrimidine, Phenyldioxane, 2-Cyclohexyl-1-phenyläthane und dergleichen. Derartige Substanzen sind dem Fachmann bekannt und viele davon sind zudem im Handel erhältlich.

Vorzugsweise enthalten die erfindungsgemässen Gemische neben den Verbindungen der Formel I eine oder mehrere Verbindungen der allgemeinen Formeln

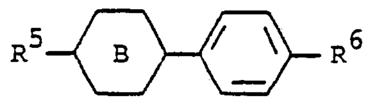

VIII

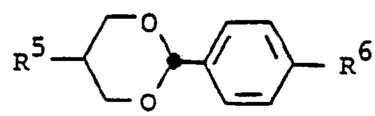

IX

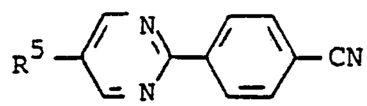

X

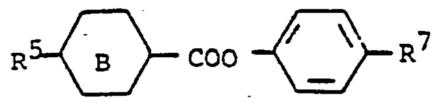

XI

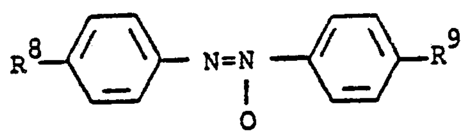

XII

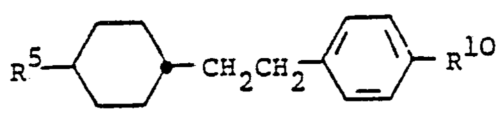

XIII

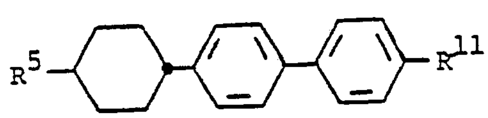

XIV

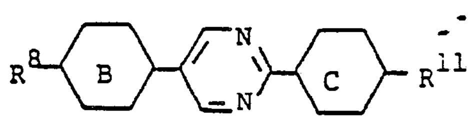

XV

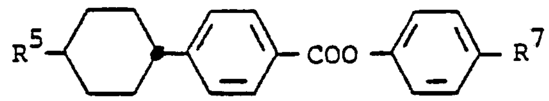 XVI

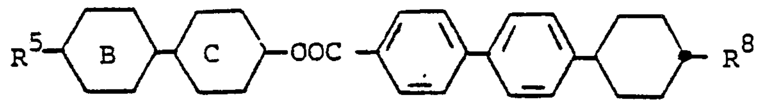 XVII

worin die Ringe B und C 1,4-Phenylen oder trans-1,4-Cyclohexylen darstellen; $R^5$ geradkettiges $C_1$-$C_7$-Alkyl, geradkettiges $C_4$-$C_7$-3E-Alkenyl oder an einem Cyclohexan- oder Dioxanring auch $C^2$-$C^7$-1E-Alkenyl bedeutet; $R^6$ geradkettiges $C_1$-$C_7$-Alkyl, geradkettiges $C_1$-$C_7$-Alkoxy, —CN oder —NCS bedeutet; $R^7$ geradkettiges $C_1$-$C_7$-Alkyl oder geradkettiges $C_1$-$C_7$-Alkoxy bezeichnet; $R^8$ und $R^9$ für geradkettiges $C_1$-$C_7$-Alkyl stehen; $R^{10}$ Cyano, Alkyl, Alkoxy, p-Alkylphenyl, p-Alkoxyphenyl, trans-4-Alkylcyclohexyl, 4'-Alkyl-4-biphenylyl, p-(trans-4-Alkylcyclohexyl)phenyl, 2-(trans-4-Alkylcyclohexyl)äthyl, p-[2-(trans-4-Alkylcyclohexyl)äthyl]phenyl oder 2-[p-(trans-4-Alkylcyclohexyl)phenyl]äthyl darstellt, und die Alkyl- und Alkoxyreste in $R^{10}$ geradkettige Reste mit 1 bis 7 Kohlenstoffatomen sind; und $R^{11}$ Cyano oder geradkettiges $C_1$-$C_7$-Alkyl bedeutet.

Die erfindungsgemässen Mischungen können ebenfalls optisch aktive Verbindungen (z. B. optisch aktive Biphenyle) und/oder dichroitische Farbstoffe (z. B. Azo-, Azoxy- oder Antrachinonfarbstoffe) enthalten. Der Anteil solcher Verbindungen wird durch die Löslichkeit, die gewünschte Ganghöhe, Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-% im Gesamtgemisch.

Die Verbindungen der Formel I, worin nicht zugleich $R^1$ 4-Pentenyl, Ring $A^1$ trans-1,4-Cyclohexylen, $X^1$ —$CH_2CH_2$—, Ring $A^2$ 1,4-Phenylen, n die Zahl 0 und $R^2$ —CN bedeuten, sind neu und ebenfalls Gegenstand der vorliegenden Erfindung.

Die obige Formel I umfasst insbesondere die Teilformeln

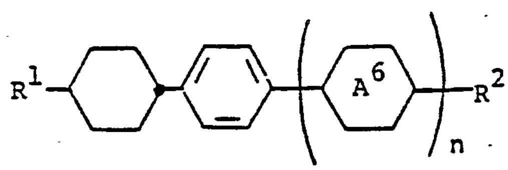 Ia

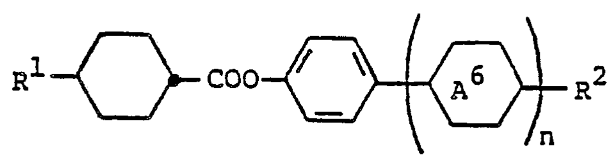 Ib

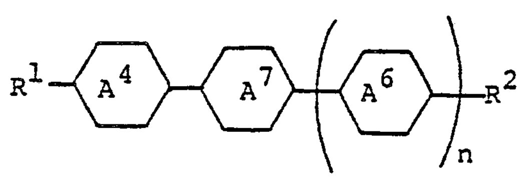 Ic

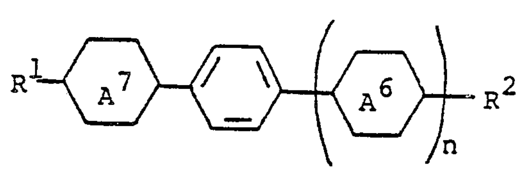 Id

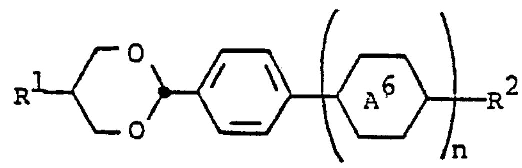

Ie

worin $R^1$, $R^2$ und n die obigen Bedeutungen haben; die Ringe $A^4$ und $A^6$ 1,4-Phenylen, 2-Fluor-1,4-phenylen oder trans-1,4-Cyclohexylen darstellen; und Ring $A^7$ einen 2,5 disubstituierten Pyrimidinring bezeichnet.

Im allgemeinen sind diejenigen Verbindungen der Formel I bevorzugt, worin $X^1$ und $X^2$ einfache Kovalenzbindungen bezeichnen oder eines dieser Symbole auch —COO— oder —OOC— bezeichnet. Ferner sind im allgemeinen diejenigen Verbindungen der Formel I bevorzugt, worin $R^1$ 4-Alkenyl, insbesondere 4Z-Alkenyl bedeutet. Vorzugsweise steht $R^1$ für geradkettige Reste mit bis zu 12 Kohlenstoffatomen, d. h. für geradkettiges 2Z-Alkenyl mit 3 bis 12 Kohlenstoffatomen oder insbesondere für geradkettiges 4-Alkenyl mit 5 bis 12 Kohlenstoffatomen, wie 4-Pentenyl, 4Z-Hexenyl, 4E-Hexenyl, 4Z-Heptenyl und 4E-Heptenyl.

$R^2$ bedeutet vorzugsweise —CN, —NCS oder geradkettige Alkyl- öder Alkoxygruppen mit 1 bis 12, insbesondere 1 bis 7 Kohlenstoffatomen. Die Reste —CN, —NCS und Alkoxy für $R^2$ stehen vorzugsweise an einem aromatischen Ring, d. h. an einem Benzol- oder Pyrimidinring.

Ein in Formel I gegebenenfalls vorhandener, lateraler Fluorsubstituent steht vorzugsweise in ortho-Stellung zu $R^2$, insbesondere wenn $R^2$ —CN oder —NCS bezeichnet. Im allgemeinen sind jedoch diejenigen Verbindungen der Formel I bevorzugt, welche keinen lateralen Fluorsubstituenten aufweisen.

Besonders bevorzugte Verbindungen der Formeln I, Ia, Ib, Ic, Id und Ie sind somit diejenigen, worin n für die Zahl 0 oder 1 steht; $X^1$ und $X^2$ einfache Kovalenzbindungen bezeichnen oder eines der Symbole $X^1$ und $X^2$ auch —COO— oder —OOC— bezeichnet; die Ringe $A^1$, $A^2$, $A^3$, $A^4$ und $A^6$ 1,4-Phenylen oder trans-1,4-Cyclohexylen darstellen oder einer der Ringe $A^1$, $A^2$ und $A^3$ auch einen 2,5-disubstituierten Pyrimidinring oder einen trans-2,5-disubstituierten m-Dioxanring darstellt; Ring $A^7$ einen 2,5-disubstituierten Pyrimidinring bezeichnet; $R^1$ geradkettiges 4-Alkenyl mit 5 bis 12 Kohlenstoffatomen bedeutet; und $R^2$ geradkettiges $C_1$-$C_{12}$-Alkyl oder an einem Benzol- oder Pyrimidinring auch —CN, —NCS oder geradkettiges $C_1$-$C_{12}$-Alkoxy bezeichnet.

Beispiele bevorzugter Verbindungen der Formel I sind:

p-[trans-4-(4-Pentenyl)cyclohexyl]benzonitril,
p-[trans-4-(4-Pentenyl)cyclohexyl]phenylisothiocyanat,
p-[trans-4-(4Z-Hexenyl)cyclohexyl]benzonitril,
p-[trans-4-(4Z-Hexenyl)cyclohexyl]phenylisothiocyanat,
1-Aethyl-4-[trans-4-(4-pentenyl)cyclohexyl]benzol,
1-Aethyl-4-(trans-4-allylcyclohexyl)benzol,
1-Aethoxy-4-[trans-4-(4-pentenyl)cyclohexyl]benzol,
1-Aethoxy-4-(trans-4-allylcyclohexyl)benzol,
1-Aethyl-4-[2-(trans-4-(4-pentenyl)cyclohexyl)äthyl]benzol,
1-Aethyl-4-[2-(trans-4-allylcyclohexyl)äthyl]benzol,
4'-(4-Pentenyl)-4-biphenylcarbonitril,
4'-[trans-4-(4-Pentenyl)cyclohexyl]-4-biphenylcarbonitril,
trans-4-(4-Pentenyl)cyclohexancarbonsäure-p-äthoxyphenylester,
p-[trans-4-(4-Pentenyl)cyclohexyl]benzoesäure-p-propylphenylester,
5-[p-(4-Pentenyl)phenyl]-2-pyrimidincarbonitril,
5-[p-(4-Pentenyl)phenyl]-2-pyrimidinylisothiocyanat,
p-[5-(4-Pentenyl)-2-pyrimidinyl]benzonitril,
p-[5-(4-Pentenyl)-2-pyrimidinyl]phenylisothiocyanat,
p-[trans-5-(4-Pentenyl)-m-dioxan-2-yl]benzonitril,
p-[trans-5-(4-Pentenyl)-m-dioxan-2-yl]phenylisothiocyanat

und die weiteren, in den Synthesebeispielen genannten Verbindungen der Formel I.

Die Verbindungen der Formel I können erfindungsgemäss dadurch hergestellt werden, dass man

a) zur Herstellung der Verbindungen der Formel I, worin $X^1$ oder $X^2$ —COO— oder —OOC— bezeichnet, eine Verbindung der allgemeinen Formel

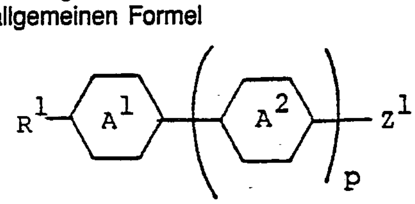

II

oder ein reaktionsfähiges Derivat hiervon mit einer Verbindung der allgemeinen Formel

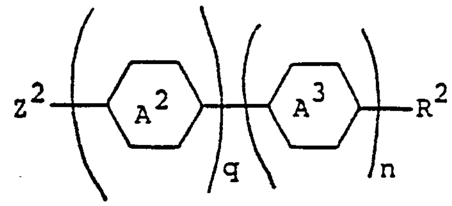

III

worin eine der Gruppen $Z^1$ und $Z^2$ —COOH und die andere —OH darstellt; einer der Indices p und q die Zahl 0 und der andere die Zahl 1 bezeichnet; und $R^1$, $R^2$, n und die Ringe $A^1$, $A^2$ und $A^3$ die obige Bedeutung haben, oder ein Reaktionsfähiges Derivat hiervon verestert, oder

b) zur Herstellung der Verbindungen der Formel I, worin einer der Ringe $A^1$, $A^2$ und $A^3$ einen trans-2,5-disubstituierten m-Dioxanring darstellt und $X^1$ und $X^2$ einfache Kovalenzbindungen oder eines dieser Symbole auch $—CH_2CH_2—$ bezeichnen, eine Verbindung der allgemeinen Formel

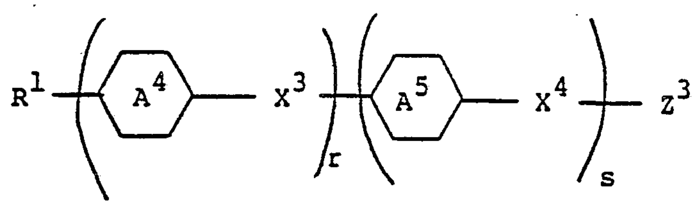

IV

mit einer Verbindung der allgemeinen Formel

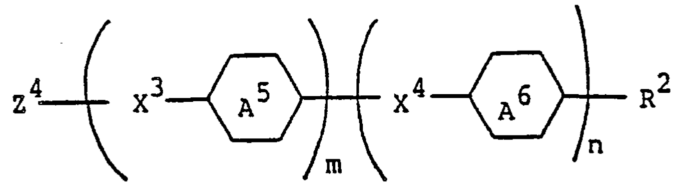

V

worin eine der Gruppen $Z^3$ und $Z^4$ $—CH(CH_2OH)_2$ und die andere —CHO darstellt; $X^3$ und $X^4$ einfache Kovalenzbindungen bezeichnen oder eines dieser Symbole auch $—CH_2CH_2—$ bezeichnet; die Ringe $A^4$, $A^5$ und $A^6$ 1,4-Phenylen, 2-Fluor-1,4-phenylen oder trans-1,4-Cyclohexylen darstellen; m, n, r und s die Zahlen 0 oder 1 bedeuten und die Summe (m + n + r + s) dieser Indices 1 oder 2 beträgt, wobei s nur dann die Zahl 1 bedeuten kann, wenn r für die Zahl 1 steht, und m nur dann die Zahl 1 bedeuten kann, wenn r für die Zahl 0 steht; und $R^1$ und $R^2$ die obigen Bedeutungen haben, umsetzt, oder

c) zur Herstellung der Verbindungen der Formel I, worin einer der Ringe $A^1$, $A^2$ und $A^3$ einen 2,5-disubstituierten Pyrimidinring darstellt und $X^1$ und $X^2$ einfache Kovalenzbindungen oder eines dieser Symbole auch $—CH_2CH_2—$ bezeichnen, eine Verbindung der allgemeinen Formel IV mit einer Verbindung der Formel V, worin eine der Gruppen $Z^3$ und $Z^4$ $H_2N—C=NH \cdot HCl$ und die andere $OHC—C=CHOR^3$ darstellt, $R^3$ Alkyl bezeichnet und $X^3$, $X^4$, $R^1$, $R^2$, m, n, r, s und die Ringe $A^4$, $A^5$ und $A^6$ die obigen Bedeutungen haben, in Gegenwart einer Base umsetzt, oder

d) zur Herstellung der Verbindungen der Formel I, worin $X^1$ und $X^2$ einfache Kovalenzbindungen oder eines dieser Symbole auch $—CH_2CH_2—$ bezeichnen, eine Verbindung der allgemeinen Formel

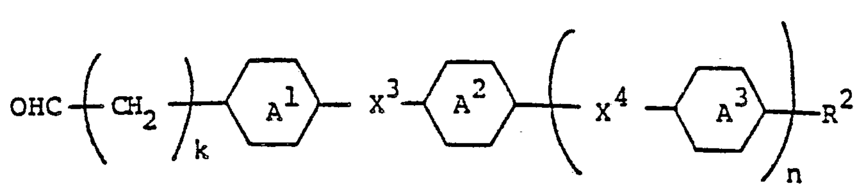

VI

worin $X^3$ und $X^4$ einfache Kovalenzbindungen bezeichnen oder eines dieser Symbole auch $—CH_2CH_2—$ bezeichnet; $R^2$, n und die Ringe $A^1$, $A^2$ und $A^3$ die obigen Bedeutungen haben; und k die Zahl 3 oder, falls Ring $A^1$ trans-1,4-Cyclohexylen darstellt, auch die Zahl 1 bezeichnet, in Gegenwart einer Base mit Alkyltriphenylphosphoniumhalogenid umsetzt, oder

e) zur Herstellung der Verbindungen der Formel I, worin $R^2$ —NCS bezeichnet eine Verbindung der

allgemeinen Formel

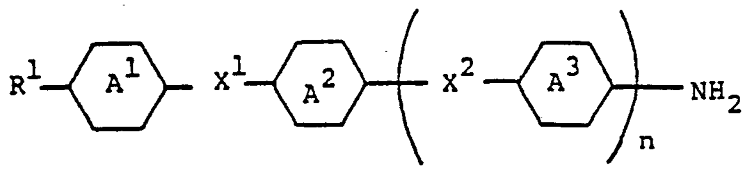 VII

worin $R^1$, $X^1$, $X^2$, n und die Ringe $A^1$, $A^2$ und $A^3$ die obigen Bedeutungen haben, in Gegenwart eines Amins mit Thiophosgen umsetzt, oder

f) zur Herstellung der Verbindungen der Formel I, worin $X^1$ und $X^2$ einfache Kovalenzbindungen oder eines dieser Symbole auch $—CH_2CH_2—$ bezeichnen, die Ringe $A^1$, $A^2$ und $A^3$ 1,4-Phenylen, 2-Fluor-1,4-phenylen oder trans-1,4-Cyclohexylen darstellen und $R^2$ primäres Alkyl bedeutet, eine Verbindung der allgemeinen Formel

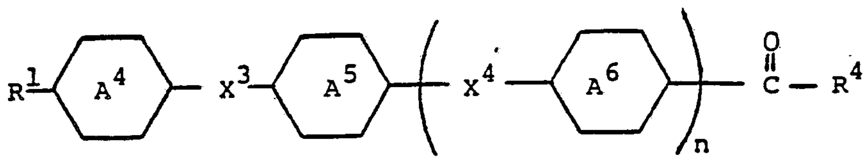 LVa

worin $X^3$ und $X^4$ einfache Kovalenzbindungen bezeichnen oder eines dieser Symbole auch $—CH_2—CH_2—$ bezeichnet; die Ringe $A^4$, $A^5$ und $A^6$ 1,4-Phenylen, 2-Fluor-1,4-phenylen oder trans-1,4-Cyclohexylen darstellen; $R^4$ Wasserstoff oder Alkyl bedeutet; und $R^1$ und n die obigen Bedeutungen haben, reduziert.

Die Veresterung der Verbindungen der Formeln II und III oder von reaktionsfähigen Derivaten hiervon (z. B. Säurechlorid, Alkalimetallalkoholat oder -phenolat) gemäss Verfahrensvariante a) kann in an sich bekannter Weise erfolgen. Die Veresterung der Säurechloride kann beispielsweise in Diäthyläther, Tetrahydrofuran, Dimethylformamid, Benzol, Toluol, Tetrachlorkohlenstoff, Pyridin und dergleichen erfolgen. Die Veresterung einer Verbindung der Formel II mit einer Verbindung der Formel III erfolgt vorzugsweise in Gegenwart von 4-(Dimethylamino)pyridin und Dicyclohexylcarbodiimid oder in Gegenwart von Oxalylchlorid und Dimethylformamid. Temperatur und Druck dieser Veresterungsreaktionen sind nicht kritisch. Im allgemeinen werden Atmosphärendruck und eine Temperatur zwischen etwa — 30 °C und der Siedetemperatur der Reaktionsgemisches angewendet.

Die Umsetzung einer Verbindung der Formel IV mit einer Verbindung der Formel V gemäss Verfahrensvariante b) kann in an sich bekannter Weise erfolgen. Anstelle des Aldehyds kann auch ein geeignetes Acetal, z. B. das Dimethylacetal, eingesetzt werden. Zweckmässigerweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure oder trockener Chlorwasserstoffsäure. Temperatur und Druck sind nicht kritisch, vorzugsweise wird jedoch bei Rückflusstemperatur und Atmosphärendruck gearbeitet.

Die Umsetzung einer Verbindung der Formel IV mit einer Verbindung der Formel V gemäss Verfahrensvariante c) kann ebenfalls in an sich bekannter Weise erfolgen. Zweckmässigerweise wird die Umsetzung in Wasser oder einem organischen Lösungsmittel (vorzugsweise einem Alkohol, wie Methanol, Aethanol, Aethylenglykol und dergleichen) in Gegenwart einer Base, vorzugsweise einem Alkalimetallalkoholat, wie Natriummethylat oder Natriumäthylat, durchgeführt. $R^3$ umfasst zweckmässigerweise Alkylreste mit 1 bis 5 Kohlenstoffatomen, wie Methyl, Aethyl, Propyl, Isopropyl und dergleichen. Temperatur und Druck sind keine kritischen Aspekte in dieser Reaktion. Im allgemeinen werden jedoch Atmosphärendruck und eine Temperatur zwischen Raumtemperatur und Rückflusstemperatur angewendet.

Die Umsetzung einer Verbindung der Formel VI mit Alkyl-triphenylphosphoniumhalogenid (vorzugsweise Alkyltriphenylphosphoniumbromid) in Gegenwart einer Base (gemäss Verfahrensvariante d) kann in an sich bekannter Weise erfolgen. Geeignete Basen sind Kalium-t-butylat, Natriummethylat, Kaliumcarbonat, Natriumhydrid, Natriumamid und dergleichen. Die Reaktion wird zweckmässig in einem inerten organischen Lösungsmittel, beispielsweise einem Aether, wie Diäthyläther, Tetrahydrofuran oder Dioxan, durchgeführt. Temperatur und Druck sind nicht kritisch; im allgemeinen wird jedoch bei Atmosphärendruck und einer Temperatur von Raumtemperatur bis Rückflusstemperatur gearbeitet.

Die Umsetzung einer Verbindung der Formel VII mit Thiophosgen in Gegenwart eines Amins gemäss Verfahrensvariante e) kann in an sich bekannter Weise erfolgen. Zweckmässigerweise wird die Umsetzung in einem inerten organischen Lösungsmittel, beispielsweise einem chlorierten oder aromatischen Kohlenwasserstoff oder einem Aether, wie Chloroform, Methylenchlorid, Benzol, Diäthyläther, Tetrahydrofuran und dergleichen, durchgeführt. Bevorzugte Basen sind Dialkylamine und insbesondere

Trialkylamine, wie Diisopropylamin, Triäthylamin und dergleichen. Temperatur und Druck sind nicht kritisch. Im allgemeinen wird jedoch bei Atmosphärendruck und einer Temperatur von —30 °C bis Rückflusstemperatur, vorzugsweise bei Raumtemperatur gearbeitet.

Die Reduktion einer Verbindung der Formel LVa gemäss Verfahrensvariante f) kann in an sich bekannter Weise erfolgen. Beispielsweise kann eine Verbindung der Formel LVa mit Hydrazin in Gegenwart eine Base (z. B. Kaliumhydroxid, Natriumäthylat, Kalium-t-butylat) in einem inerten organischen Lösungsmittel, wie Dimethylformamid, Aethanol, Diäthylenglykol oder Triäthylenglykol, umgesetzt und anschliessend das gebildete Hydrazon bei erhöhter Temperatur zersetzt werden. Eine bevorzugte Variante ist die Umsetzung nach dem Huang-Minlon-Verfahren, d. h. Erhitzen der Verbindung der Formel LVa unter Rückfluss in einem hochsiedenden mit Wasser mischbaren Lösungsmittel (z. B. Diäthylenglykol oder Triäthylenglykol), zusammen mit Hydrazinhydrat und Kaliumhydroxid, anschliessendes Abdestillieren des Wassers bis zur Zersetzung des Hydrazons, und weiteres Kochen unter Rückfluss, bis die Reduktion beendet ist. Ferner können die Verbindungen der Formel LVa durch Erwärmen mit amalgamiertem Zink und Salzsäure reduziert werden ; gewünschtenfalls kann dem Reaktionsgemisch ein organisches Lösungsmittel, wie Aethanol, Essigsäure, Dioxan oder Toluol, zugesetzt werden.

Die Verbindungen der obigen Formeln III und V sind bekannte oder Analoge bekannter Verbindungen.

Die Verbindungen der Formel LVa sind neu und ebenfalls Gegenstand der vorliegenden Erfindung. Sie können in an sich bekannter Weise aus den entsprechenden Verbindungen der Formel I, worin $R^2$ Cyano bedeutet, erhalten werden. Beispielsweise werden die Verbindungen der Formel LVa, worin $R^4$ Wasserstoff bedeutet, erhalten durch Reduktion der Cyano-Verbindungen mit Diisobutylaluminiumhydrid und die Verbindungen der Formel LVa, worin $R^4$ Alkyl bedeutet, durch Umsetzung der Cyano-Verbindungen mit Alkylmagnesiumhalogenid und anschliessende Hydrolyse.

Die Verbindungen der Formeln II, IV, VI und VII sind ebenfalls neu. Die Herstellung dieser Verbindungen und weitere Alternativen zur Herstellung der Verbindungen der Formel I werden anhand repräsentativer Beispiele in den folgenden Reaktionsschemata 1-8 veranschaulicht, worin $R^1$ und $R^2$ die obigen Bedeutungen haben, Ring $A^5$ 1,4-Phenylen, 2-Fluor-1,4-phenylen oder trans-1,4-Cyclohexylen darstellt, R und $R^4$ Wasserstoff oder Alkyl bezeichnen und Ts für p-Tosyl steht.

(Siehe Schemen Siehe 9 ff.)

Die Verbindung der Formel XX kann in analoger Weise zu Schema 2 zu entsprechenden Alkenyl-Verbindungen umgesetzt werden.

Alkenylphenole können beispielsweise durch Umsetzung von p-Hydroxybenzaldehyl mit (2-Methoxyäthoxy)methylchlorid (Schutz der Hydroxygruppe), anschliessende Einführung der Alkenylgruppe nach der in Schema 1 beschriebenen Methode und Abspaltung der Schutzgruppe mittels Zinkbromid erhalten werden.

4'-Cyano-4-biphenylcarboxaldehyd kann beispielsweise dadurch erhalten werden, dass man 4-Brombiphenyl zuerst mit $Cl_2CHOCH_3$ und Titantetrachlorid und dann mit Wasser umsetzt und den erhaltenen Brom-aldehyd mit Kupfer-(I)-cyanid in Dimethylformamid umsetzt. Der erhaltene Cyanoaldehyd kann dann beispielsweise in analoger Weise zu den in Schema 3 beschriebenen Methoden weiter umgesetzt werden.

Die Einführung der Alkenylgruppe gemäss XXXV → XXXVI, XXXVIII → XXXIX und IL → XLIII kann in analoger Weise zu der in Schema 1 und 2 dargestellten Methode erfolgen. Weitere Alternativen, welche insbesondere bei der Einführung von 4-Alkenylgruppen, einen kürzeren Syntheseweg ermöglichen sind in den nachstehenden Synthesebeispielen 8-10 illustriert.

In obigen Reaktionen werden in einigen Fällen Gemische von cis/trans-isomeren Cyclohexanderivaten erhalten, welche jedoch in an sich bekannter Weise, z. B. durch Chromatographie und/oder Kristallisation, getrennt werden können. Ferner werden in einigen Fällen E/Z-Gemische von Alkenylverbindungen erhalten, insbesondere bei der Einführung der Alkenylgruppe durch Wittig-Reaktion. Diese können ebenfalls nach an sich bekannten Methoden getrennt werden, z. B. durch Chromatographie. Hierbei hat sich insbesondere mit Silbernitrat imprägniertes Kieselgel als vorteilhaft erwiesen. Gewünschtenfalls kann das unerwünschte Alkenylisomere in das gewünschte Isomere übergeführt werden, beispielsweise durch Umsetzung mit Benzolsulfinsäure oder via das Epoxid, z. B. durch Umsetzung mit m-Chlorperbenzoesäure in Gegenwart von Kaliumcarbonat, Halogenierung des Epoxids mit Triphenylphosphin-Brom in Benzol und Reduktion des Halogenids mit Zink in Eisessig.

Weitere Ausgangsmaterialien können in analoger Weise zu den beschriebenen Methoden oder nach weiteren, dem Fachmann geläufigen Methoden hergestellt werden.

Die Verbindungen der Formeln VIII-XI, XIII, XIV, XVI und XVII, worin $R^5$ 1E-Alkenyl oder 3E-Alkenyl bedeutet sind ebenfalls neu. Sie können in analoger Weise zu den für die Verbindungen der Formel I beschriebenen Methoden hergestellt werden.

Beispiele bevorzugter erfindungsgemässer Gemische sind die folgenden Mischungen 1-17. $V_{10}$ und $V_{50}$ bezeichnen die Spannung für 10 % bzw. 50 % Transmission (in einer Drehzelle bei 0° Kippwinkel), $p_o = (V_{50}—V_{10})/V_{10}$ ist ein Mass für die Steilheit der Transmissionskurve und $k_{11}$ (splay) und $k_{33}$ (bend)

Schema 1

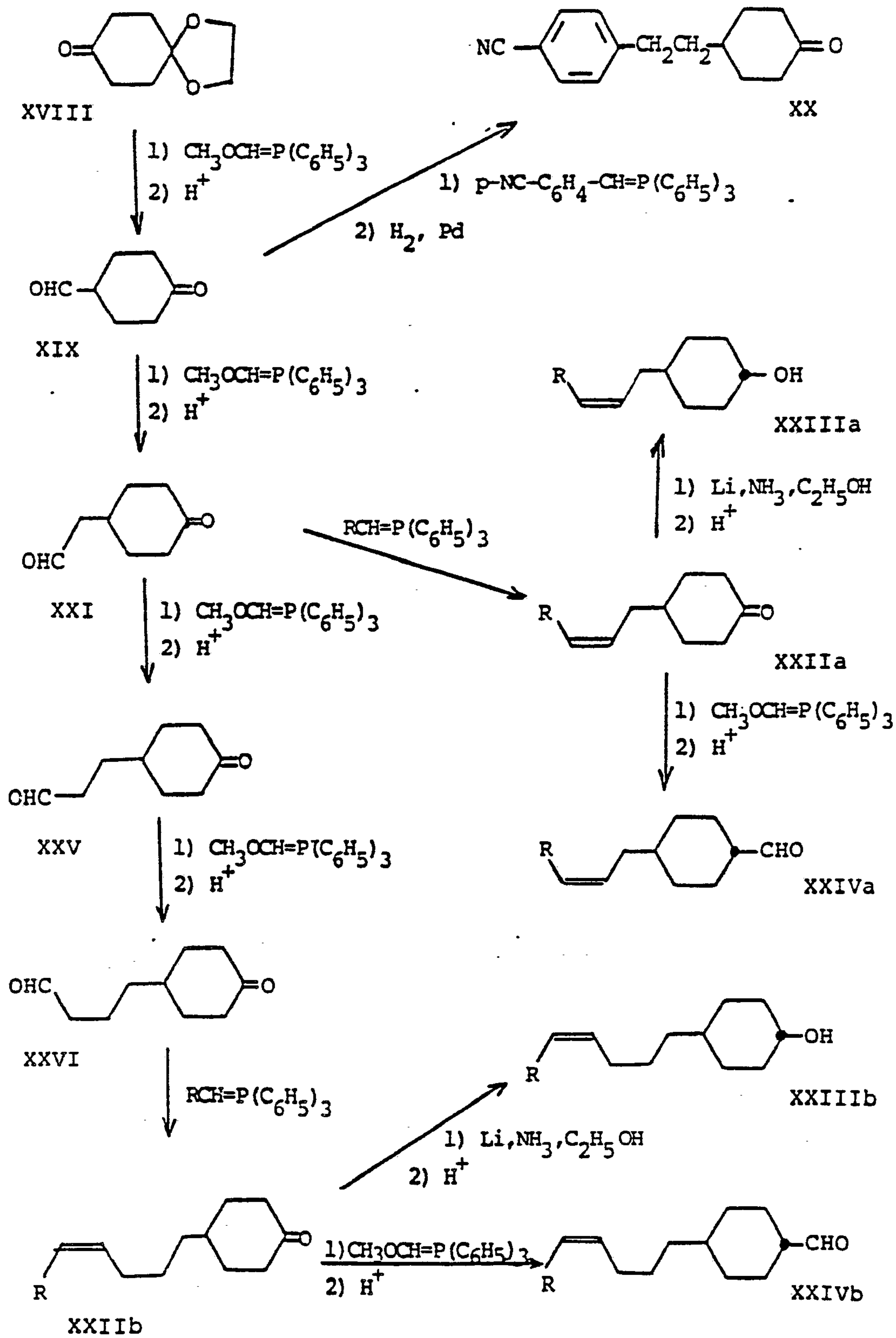
XVIII
1) $CH_3OCH=P(C_6H_5)_3$
2) $H^+$
1) $p-NC-C_6H_4-CH=P(C_6H_5)_3$
2) $H_2$, Pd
XX
XIX
1) $CH_3OCH=P(C_6H_5)_3$
2) $H^+$
XXIIIa
1) Li, $NH_3$, $C_2H_5OH$
2) $H^+$
XXI
$RCH=P(C_6H_5)_3$
1) $CH_3OCH=P(C_6H_5)_3$
2) $H^+$
XXIIa
1) $CH_3OCH=P(C_6H_5)_3$
2) $H^+$
XXV
1) $CH_3OCH=P(C_6H_5)_3$
2) $H^+$
XXIVa
XXVI
$RCH=P(C_6H_5)_3$
XXIIIb
1) Li, $NH_3$, $C_2H_5OH$
2) $H^+$
1) $CH_3OCH=P(C_6H_5)_3$
2) $H^+$
XXIIb
XXIVb

Schema 2

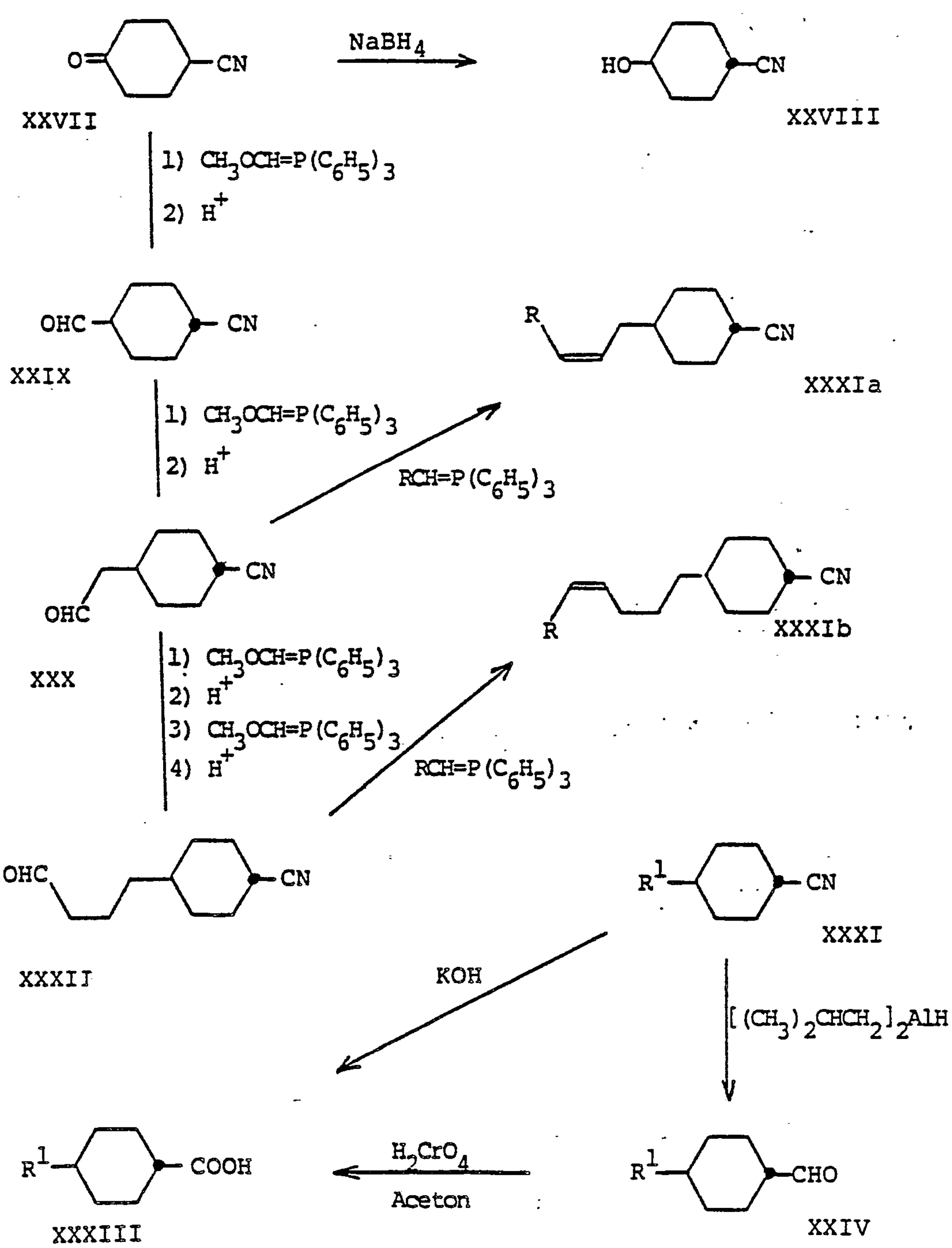


XXVII

XXVIII

XXIX

XXXIa

XXX

XXXIb

XXXII

XXXI

XXXIII

XXIV

Schema 3

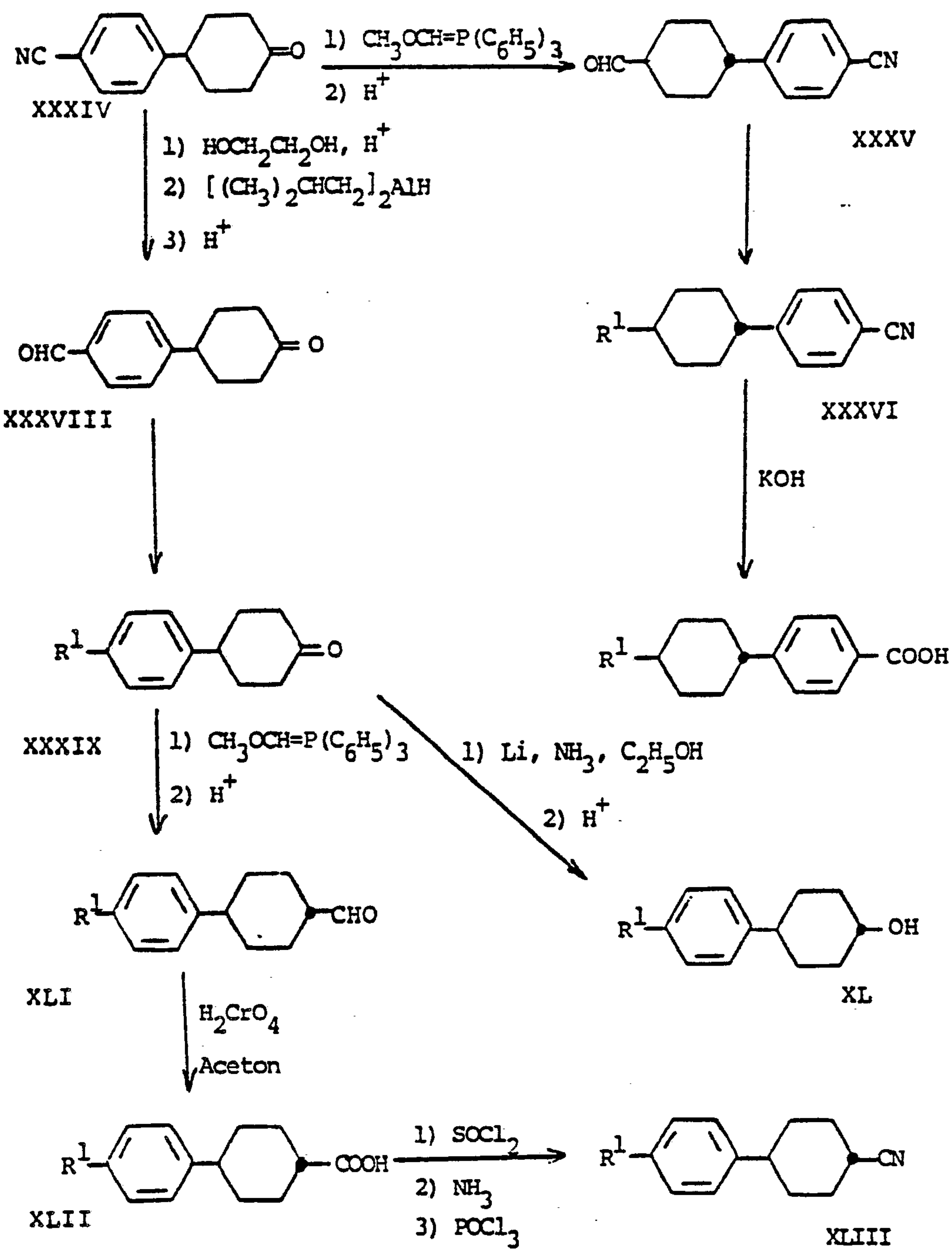


XXXIV, XXXV, XXXVIII, XXXVI, XXXIX, XLI, XL, XLII, XLIII

Schema 4

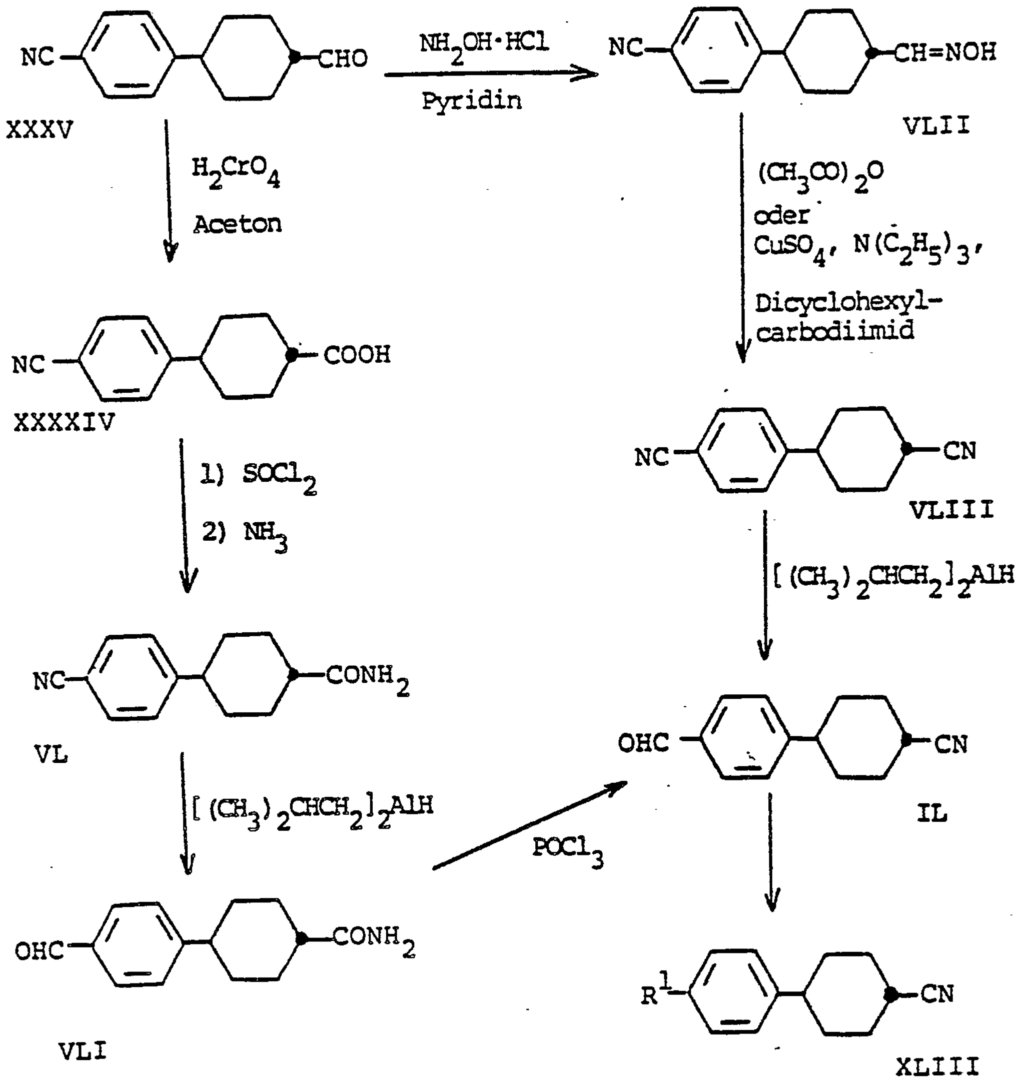
NH2OH·HCl
Pyridin
H2CrO4
Aceton
(CH3CO)2O
oder
CuSO4, N(C2H5)3,
Dicyclohexyl-
carbodiimid
1) SOCl2
2) NH3
[(CH3)2CHCH2]2AlH
[(CH3)2CHCH2]2AlH
POCl3

Schema 5

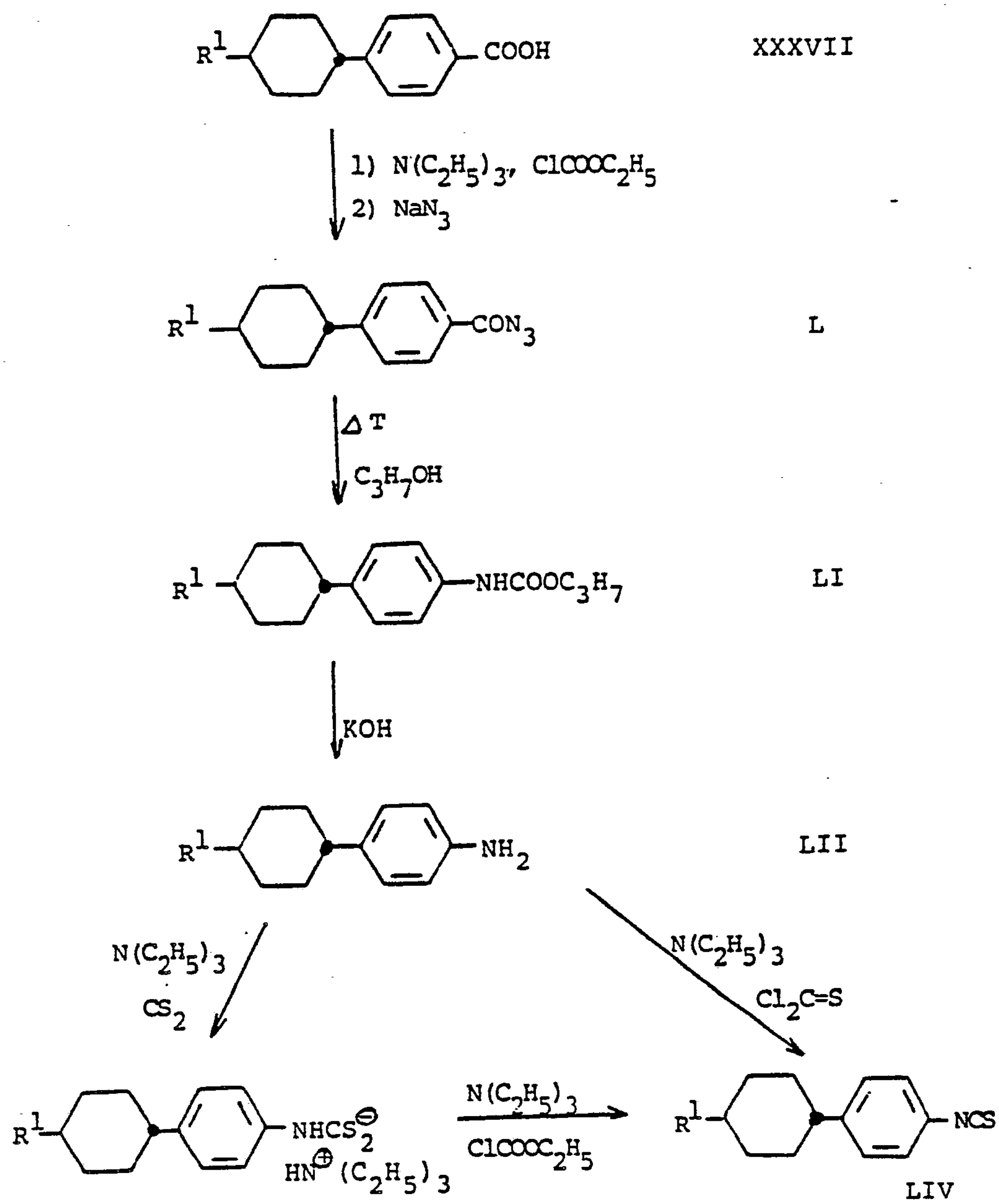


XXXVII

L

LI

LII

LIII

LIV

Schema 6

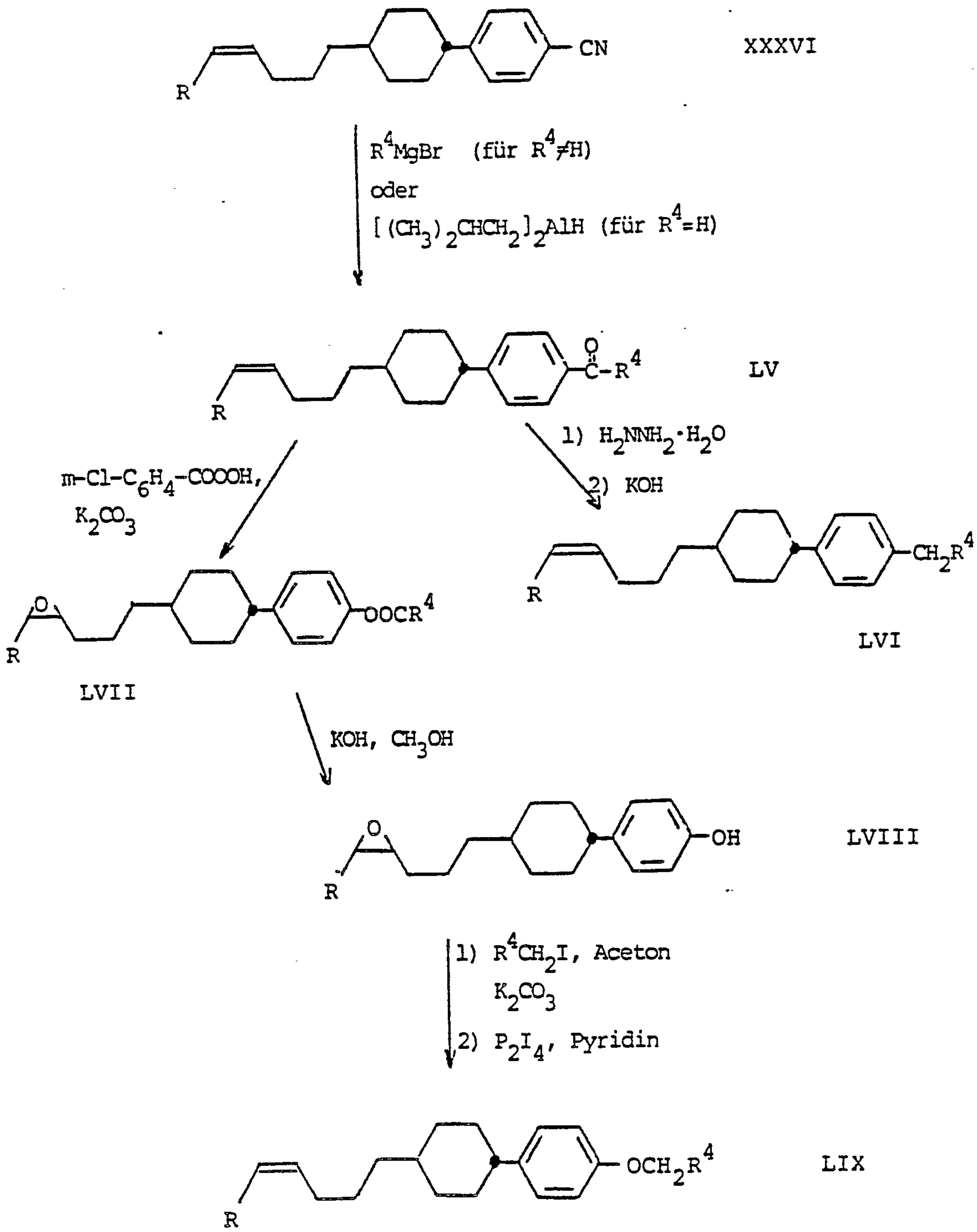


XXXVI

LV

LVI

LVII

LVIII

LIX

Schema 7

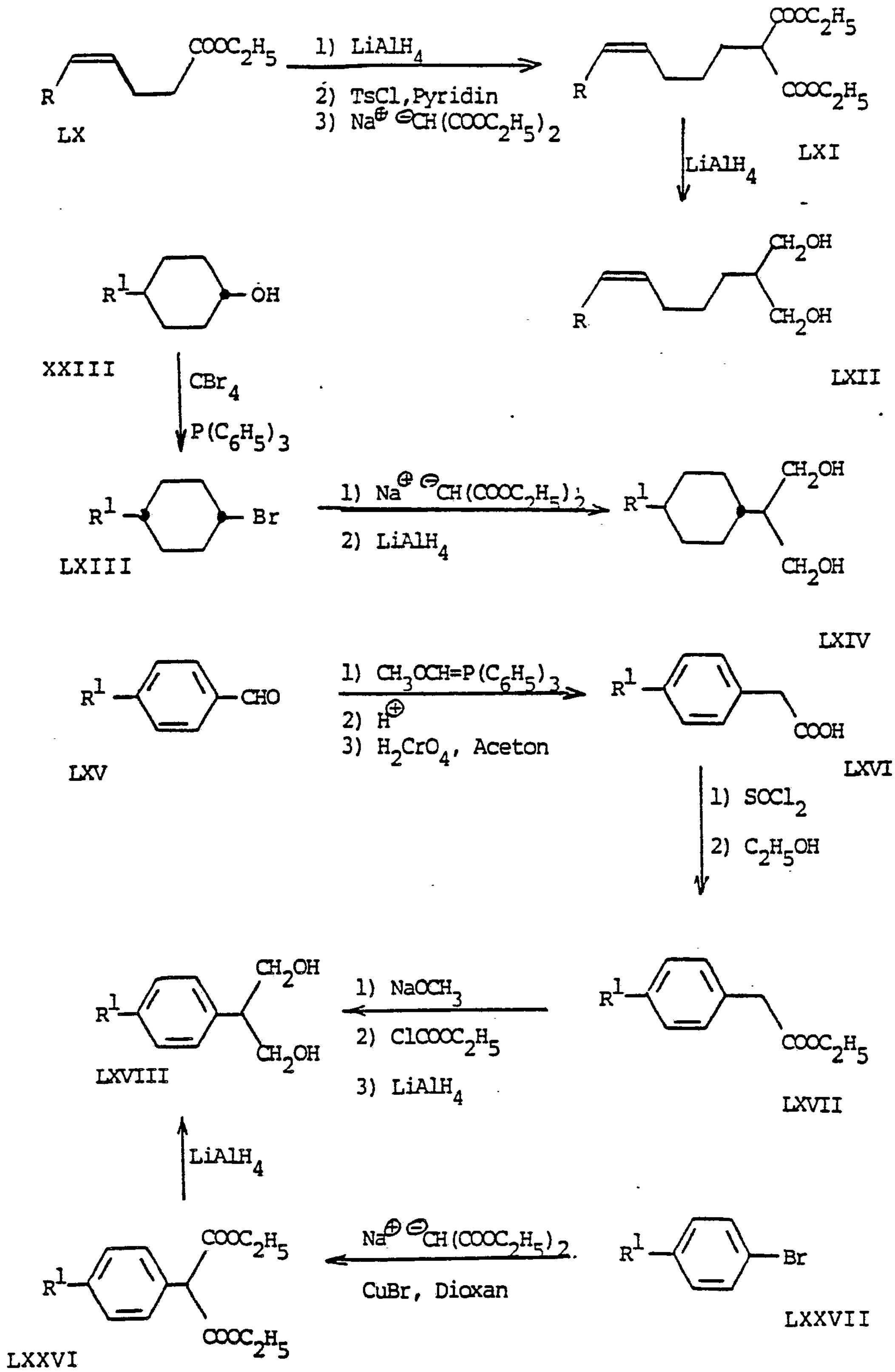


LX, LXI, XXIII, LXII, LXIII, LXIV, LXV, LXVI, LXVIII, LXVII, LXXVI, LXXVII

Schema 8

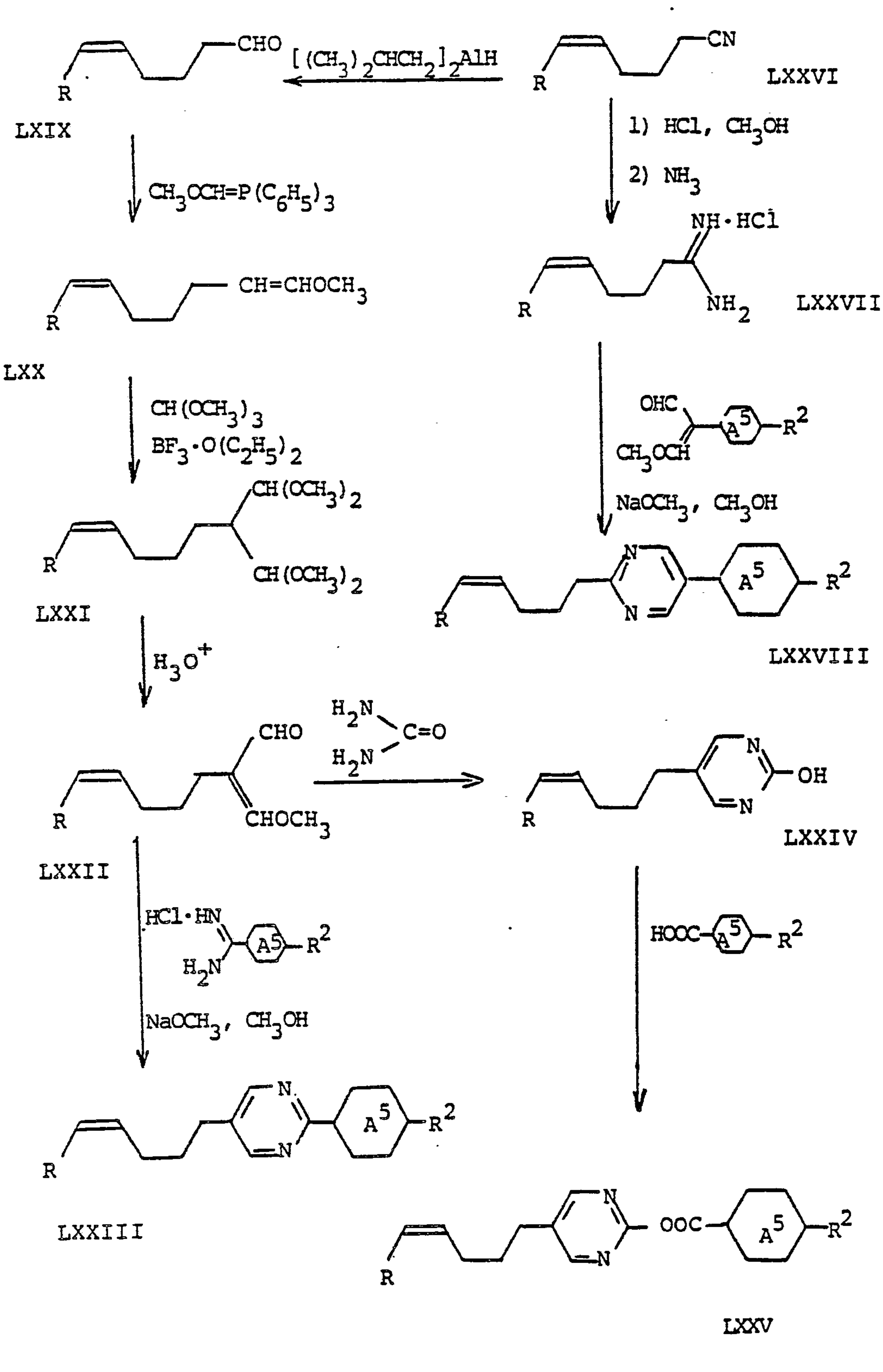


LXIX, LXX, LXXI, LXXII, LXXIII, LXXIV, LXXV, LXXVI, LXXVII, LXXVIII

sind elastische Konstanten. $t_{on}$ und $t_{off}$ bezeichnen die Einschaltzeit bzw. Ausschaltzeit und $\Delta n$ bezeichnet die optische Anisotropie. Sofern nicht anders angegeben, wurden die Messungen bei 22 °C durchgeführt.

Mischung 1

7,8 Gew.-% p-(5-Butyl-2-pyrimidinyl)benzonitril,
2,6 Gew.-% p-(5-Pentyl-2-pyrimidinyl)benzonitril,
5,0 Gew.-% p-(5-Heptyl-2-pyrimidinyl)benzonitril,
7,0 Gew.-% 4-Aethyl-1-[2-(trans-4-propylcyclohexyl)äthyl]benzol,
9,3 Gew.-% trans-4-Butylcyclohexancarbonsäure-p-äthoxyphenylester,
11,5 Gew.-% trans-4-Butylcyclohexancarbonsäure-p-pentyloxyphenylester,
8,5 Gew.-% trans-4-Pentylcyclohexancarbonsäure-p-methoxyphenylester,
14,4 Gew.-% trans-4-Pentylcyclohexancarbonsäure-p-propyloxyphenylester,
3,7 Gew.-% 5-(p-Butylphenyl)-2-(p-pentylphenyl)pyrimidin,
6,5 Gew.-% 5-(trans-4-Aethylcyclohexyl)-2-(p-pentylphenyl)pyrimidin,
4,5 Gew.-% p-[5-(trans-4-Aethylcyclohexyl)-2-pyrimidinyl]benzonitril,
3,4 Gew.-% p-[5-trans-4-Pentylcyclohexyl)-2-pyrimidinyl]benzonitril,
7,4 Gew.-% 1-[2-(trans-4-Butylcyclohexyl)äthyl]-4-(trans-4-pentylcyclohexyl)benzol,
8,4 Gew.-% p-[trans-4-(4-Pentenyl)cyclohexyl]benzonitril ;
Smp. < — 10 °C, Klp. 71 °C, nematisch ; $V_{10}$ = 1,729 V, $p_o$ = 0,114, $k_{33}/k_{11}$ = 0,87.

Mischung 2

8,2 Gew.-% p-(5-Butyl-2-pyrimidinyl)benzonitril,
2,7 Gew.-% p-(5-Pentyl-2-pyrimidinyl)benzonitril,
5,2 Gew.-% p-(5-Heptyl-2-pyrimidinyl)benzonitril,
6,6 Gew.-% 4-Aethyl-1-[2-(trans-4-propylcyclohexyl)äthyl]benzol,
9,7 Gew.-% trans-4-Butylcyclohexancarbonsäure-p-äthoxyphenylester,
12,0 Gew.-% trans-4-Butylcyclohexancarbonsäure-p-pentyloxyphenylester,
8,9 Gew.-% trans-4-Pentylcyclohexancarbonsäure-p-methoxyphenylester,
15,1 Gew.-% trans-4-Pentylcyclohexancarbonsäure-p-propyloxyphenylester,
3,8 Gew.-% 5-(p-Butylphenyl)-2-(p-pentylphenyl)pyrimidin,
6,6 Gew.-% 5-(trans-4-Aethylcyclohexyl)-2-(p-pentylphenyl)pyrimidin,
4,7 Gew.-% p-[5-(trans-4-Aethylcyclohexyl)-2-pyrimidinyl]benzonitril,
5,0 Gew.-% 4-[2-(trans-4-Butylcyclohexyl)äthyl]-4'-(trans-4-pentylcyclohexyl)biphenyl
11,5 Gew.-% p-[trans-4-(4-Pentenyl)cyclohexyl]benzonitril ;
Smp. < — 20 °C, Klp. 63,6 °C, nematisch ; $V_{10}$ = 1,496 V, $p_o$ = 0,120, $k_{33}/k_{11}$ = 0,88.

Mischung 3

11,4 Gew.-% p-(5-Butyl-2-pyrimidinyl)benzonitril,
3,5 Gew.-% p-(5-Pentyl-2-pyrimidinyl)benzonitril,
5,5 Gew.-% p-(5-Butyl-2-pyrimidinyl)phenylisothiocyanat,
14,6 Gew.-% trans-4-Butylcyclohexancarbonsäure-p-äthoxyphenylester,
17,8 Gew.-% trans-4-Butylcyclohexancarbonsäure-p-pentyloxyphenylester,
12,6 Gew.-% trans-4-Pentylcyclohexancarbonsäure-p-methoxyphenylester,
4,5 Gew.-% p-[5-(trans-4-Aethylcyclohexyl)-2-pyrimidinyl]phenylisothiocyanat,
16,5 Gew.-% p-[5-trans-4-(4-Pentylcyclohexyl)cyclohexyl]benzonitril,
13,6 Gew.-% 4'-[trans-4-(4-Pentenyl)cyclohexyl]-4-biphenylcarbonitril ;
Smp. < — 25 °C, Klp. 65 °C, nematisch ; $V_{10}$ = 1,530 V, $p_o$ = 0,121, $k_{33}/k_{11}$ = 1,03.

Mischung 4

15,2 Gew.-% p-(trans-4-Pentylcyclohexyl)benzonitril,
2,7 Gew.-% 4-Aethoxy-1-(trans-4-propylcyclohexyl)benzol,
7,7 Gew.-% p-(5-Butyl-2-pyrimidinyl)benzonitril,
4,5 Gew.-% p-(5-Heptyl-2-pyrimidinyl)benzonitril,
6,3 Gew.-% 4-Aethyl-1-[2-(trans-4-propylcyclohexyl)äthyl]benzol,
9,1 Gew.-% trans-4-Butylcyclohexancarbonsäure-p-äthoxyphenylester,
8,3 Gew.-% trans-4-Pentylcyclohexancarbonsäure-p-methoxyphenylester,
13,5 Gew.-% trans-4-Pentylcyclohexancarbonsäure-p-propoxyphenylester,
5,4 Gew.-% 4-Cyano-4'-(trans-4-pentylcyclohexyl)biphenyl,
5,5 Gew.-% p-[5-(trans-4-Aethylcyclohexyl)-2-pyrimidinyl]benzonitril,
11,8 Gew.-% 1-[2-(trans-4-Butylcyclohexyl)äthyl]-4-(trans-4-pentylcyclohexyl)benzol,

10,0 Gew.-% p-[trans-4-(2Z-Pentenyl)cyclohexyl]benzonitril ;
Smp. < — 20 °C, Klp. 60,1 °C, nematisch ; $V_{10}$ = 1,512 V, $p_o$ = 0,125, $k_{33}/k_{11}$ = 1,21.

Mischung 5

12,0 Gew.-% p-(trans-4-Pentylcylohexyl)benzonitril,
6,3 Gew.-% p-(5-Butyl-2-pyrimidinyl)benzonitril,
1,7 Gew.-% p-(5-Pentyl-2-pyrimidinyl)benzonitril,
3,1 Gew.-% p-(5-Heptyl-2-pyrimidinyl)benzonitril,
10,0 Gew.-% 4-Aethyl-1-[2-(trans-4-propylcyclohexyl)äthyl]benzol,
7,5 Gew.-% trans-4-Butylcyclohexancarbonsäure-p-äthoxyphenylester,
9,3 Gew.-% trans-4-Butylcyclohexancarbonsäure-p-pentoxyphenylester,
6,8 Gew.-% trans-4-Pentylcyclohexancarbonsäure-p-methoxyphenylester,
11,1 Gew.-% trans-4-Pentylcyclohexancarbonsäure-p-propoxyphenylester,
8,6 Gew.-% 5-(trans-4-Aethylcyclohexyl)-2-(p-pentylphenyl)pyrimidin,
3,7 Gew.-% p-[5-(p-Butylphenyl)-2-pyrimidinyl]benzonitril,
3,9 Gew.-% p-[5-(trans-4-Aethylcyclohexyl)-2-pyrimidinyl]benzonitril,
6,0 Gew.-% 1-[2-(trans-4-Butylcyclohexyl)äthyl]-4-(trans-4-pentylcyclohexyl)benzol,
10,0 Gew.-% p-[trans-4-(4-Pentenyl)cyclohexyl]benzonitril ;
Smp. < — 20 °C, Klp. 64 °C, nematisch ; $V_{10}$ = 1,529 V, $p_o$ = 0,124, $k_{33}/k_{11}$ = 1,03.

Mischung 6

5,0 Gew.-% p-[trans-4-(1E-Pentenyl)cyclohexyl]phenylisothiocyanat,
8,3 Gew.-% p-(5-Butyl-2-pyrimidinyl)benzonitril,
2,2 Gew.-% p-(5-Pentyl-2-pyrimidinyl)benzonitril,
4,9 Gew.-% p-(5-Heptyl-2-pyrimidinyl)benzonitril,
4,0 Gew.-% 4-Aethyl-1-[2-(trans-4-propylcyclohexyl)äthyl]benzol,
9,7 Gew.-% trans-4-Butylcyclohexancarbonsäure-p-äthoxyphenylester,
12,1 Gew.-% trans-4-Butylcyclohexancarbonsäure-p-pentoxyphenylester,
8,8 Gew.-% trans-4-Pentylcyclohexancarbonsäure-p-methoxyphenylester,
15,4 Gew.-% trans-4-Pentylcyclohexancarbonsäure-p-propoxyphenylester,
4,0 Gew.-% 5-(p-Butylphenyl)-2-(p-pentylphenyl)pyrimidin,
7,0 Gew.-% 5-(trans-4-Aethylcyclohexyl)-2-(p-pentylphenyl)pyrimidin,
6,8 Gew.-% p-[5-(trans-4-Aethylcyclohexyl)-2-pyrimidinyl]benzonitril,
11,8 Gew.-% p-[trans-4-(4-Pentenyl)cyclohexyl]benzonitril ;
Smp. ca. — 15 °C, Klp. 64,3 °C, nematisch ; $V_{10}$ = 1,580 V, $p_o$ = 0,117, $k_{33}/k_{11}$ = 0,88.

Mischung 7

33,40 Gew.-% 4'-Pentyl-4-biphenylcarbonitril,
4,68 Gew.-% 4'-Heptyl-4-biphenylcarbonitril,
14,70 Gew.-% p-(5-Butyl-2-pyrimidinyl)benzonitril,
5,20 Gew.-% p-(5-Pentyl-2-pyrimidinyl)benzonitril,
10,00 Gew.-% p-(5-Heptyl-2-pyrimidinyl)benzonitril,
7,00 Gew.-% 4-Aethyl-1-[2-(trans-4-propylcyclohexyl)äthyl]benzol,
6,32 Gew.-% trans-4-Butylcyclohexancarbonsäure-p-äthoxyphenylester,
4,00 Gew.-% 5-(p-Butylphenyl)-2-(p-pentylphenyl)pyrimidin,
6,00 Gew.-% 5-(trans-4-Aethylcyclohexyl)-2-(p-pentylphenyl)pyrimidin,
8,70 Gew.-% 4'-[trans-4-(4-Pentenyl)cyclohexyl]-4-biphenylcarbonitril ;
Smp. < — 25 °C, Klp. 61 °C, nematisch ; $V_{10}$ = 1,31 V, $p_o$ = 0,132, $k_{33}/k_{11}$ = 1,14 ; ΔN = 0,183.

Mischung 8

11,38 Gew.-% p-(5-Butyl-2-pyrimidinyl)benzonitril,
3,44 Gew.-% p-(5-Pentyl-2-pyrimidinyl)benzonitril,
6,70 Gew.-% p-(5-Heptyl-2-pyrimidinyl)benzonitril,
13,56 Gew.-% trans-4-Butylcyclohexancarbonsäure-p-äthoxyphenylester,
17,80 Gew.-% trans-4-Butylcyclohexancarbonsäure-p-pentyloxyphenylester,
12,51 Gew.-% trans-4-Pentylcyclohexancarbonsäure-p-methoxyphenylester,
5,71 Gew.-% p-[5-(trans-4-Pentylcyclohexyl)-2-pyrimidinyl]benzonitril,
16,49 Gew.-% p-[trans-4-(4-Pentenyl)cyclohexyl]benzonitril,
12,41 Gew.-% 4'-[trans-4-(4-Pentenyl)cyclohexyl]-4-biphenylcarbonitril ;
Smp. < — 25 °C, Klp. 69 °C, nematisch ; $V_{10}$ = 1,39 V, $p_o$ = 0,124, $k_{33}/k_{11}$ = 1,09 ; Δn = 0,129.

Mischung 9

11,49 Gew.-% p-(5-Butyl-2-pyrimidinyl)benzonitril,
3,48 Gew.-% p-(5-Heptyl-2-pyrimidinyl)benzonitril,
6,76 Gew.-% p-(5-Heptyl-2-pyrimidinyl)benzonitril,
13,68 Gew.-% trans-4-Butylcyclohexancarbonsäure-p-äthoxyphenylester,
17,97 Gew.-% trans-4-Butylcyclohexancarbonsaure-p-pentyloxyphenylester,
5,76 Gew.-% p-[5-(trans-4-Pentylcyclohexyl)-2-pyrimidinyl]benzonitril,
16,65 Gew.-% p-[trans-4-(4-Pentenyl)cyclohexyl]benzonitril,
11,68 Gew.-% p-[2-(trans-4-Pentenyl)cyclohexyl)]äthylbenzonitril,
12,53 Gew.-% 4'-[trans-4-(4-Pentenyl)cyclohexyl]-4-biphenylcarbonitril ;
Smp. < — 25 °C, Klp. 63 °C, nematisch ; $V_{10}$ = 1,318 V, $p_o$ = 0,121, $k_{33}/k_{11}$ = 1,11 ; Δn = 0,127.

Mischung 10

11,59 Gew.-% p-(5-Butyl-2-pyrimidinyl)benzonitril,
3,51 Gew.-% p-(5-Pentyl-2-pyrimidinyl)benzonitril,
6,82 Gew.-% p-(5-Heptyl-2-pyrimidinyl)benzonitril,
10,86 Gew.-% p-(trans-5-Pentyl-m-dioxan-2-yl)benzonitril,
13,81 Gew.-% trans-4-Butylcyclohexancarbonsäure-p-äthoxyphenylester,
18,14 Gew.-% trans-4-Butylcyclohexancarbonsäure-p-pentyloxyphenylester,
5,82 Gew.-% p-[5-(trans-4-Pentylcyclohexyl)-2-pyrimidinyl]benzonitril,
16,80 Gew.-% p-[trans-4-Pentenyl)cyclohexyl]benzonitril,
12,65 Gew.-% 4'-[trans-4-(4-Pentenyl)cyclohexyl]-4-biphenylcarbonitril ;
Smp. < — 25 °C, Klp. 68 °C, nematisch ; $V_{10}$ = 1,272 V, $p_o$ = 0,129, $k_{33}/k_{11}$ = 1,13 ; Δn = 0,135.

Mischung 11

11,31 Gew.-% p-(5-Butyl-2-pyrimidinyl)benzonitril,
3,42 Gew.-% p-(5-Pentyl-2-pyrimidinyl)benzonitril,
5,71 Gew.-% p-(5-Heptyl-2-pyrimidinyl)benzonitril,
13,47 Gew.-% trans-4-Butylcyclohexancarbonsäure-p-äthoxyphenylester,
17,69 Gew.-% trans-4-Butylcyclohexancarbonsäure-p-pentyloxyphenylester,
5,67 Gew.-% p-[5-(trans-4-Pentylcyclohexyl)-2-pyrimidinyl]benzonitril,
16,39 Gew.-% p-[trans-4-(4-Pentenyl)cyclohexyl]benzonitril,
14,00 Gew.-% trans-4-(4-Pentenyl)cyclohexancarbonsäure-p-äthoxyphenylester,
12,34 Gew.-% 4'-[trans-4-(4-Pentenyl)cyclohexyl]-4-biphenylcarbonitril ;
Smp. < — 25 °C, Klp. 66 °C, nematisch ; $V_{10}$ = 1,342 V, $p_o$ = 0,119, $k_{33}/k_{11}$ = 1,01 ; Δn = 0,128.

Mischung 12

10,70 Gew.-% p-(5-Butyl-2-pyrimidinyl)benzonitril,
3,02 Gew.-% p-(5-Pentyl-2-pyrimidinyl)benzonitril,
6,71 Gew.-% p-(5-Heptyl-2-pyrimidinyl)benzonitril,
12,81 Gew.-% trans-4-Butylcyclohexancarbonsäure-p-äthoxyphenylester,
16,67 Gew.-% trans-4-Butylcyclohexancarbonsäure-p-pentyloxyphenylester,
11,90 Gew.-% trans-4-Pentycyclohexancarbonsäure-p-methoxyphenylester,
5,01 Gew.-% p-[5-(trans-4-Pentylcyclohexyl)-2-pyrimidinyl]benzonitril,
10,00 Gew.-% p-[trans-4-(4-Pentenyl)cyclohexyl]benzonitril,
10,30 Gew.-% p-[trans-4-(4-Pentenyl)cyclohexyl]phenylisothiocyanat,
12,88 Gew.-% 4'-[trans-4-(4-Pentenyl)cyclohexyl]-4-biphenylcarbonitril ;
Smp. < — 20 °C, Klp. 67 °C, nematisch ; $V_{10}$ = 1,44 V, $p_o$ = 0,118.

Mischung 13

3,50 Gew.-% 4'-Propyl-4-biphenylcarbonitril,
17,10 Gew.-% 4'-Pentyl-4-biphenylcarbonitril,
14,47 Gew.-% 4-Aethoxy-1-[2-(trans-4-propylcyclohexyl)äthyl]benzol,
19,94 Gew.-% 4-Aethoxy-1-[2-(trans-4-pentylcyclohexyl)äthyl]benzol,
5,15 Gew.-% 4''-Pentyl-4-cyano-p-terphenyl,
5,25 Gew.-% 4'-(trans-4-Pentylcyclohexyl)-4-biphenylcarbonitril,
11,51 Gew.-% 1-[2-(trans-4-Butylcyclohexyl)äthyl]-4-(trans-4-pentylcyclohexyl)benzol,
5,38 Gew.-% 4'-[2-(trans-4-Butylcyclohexyl)äthyl]-4-(trans-4-pentylcyclohexyl)biphenyl,
7,70 Gew.-% 4'-[2-(trans-4-Butylcyclohexyl)äthyl]-4-(trans-4-pentylcyclohexyl)-1,1'-äthylendibenzol,
10,00 Gew.-% trans-4-(4-Pentenyl)cyclohexancarbonsäure-m-fluor-p-cyanophenylester ;

Smp. < — 20 °C, Klp. 81 °C, nematisch ; $V_{10}$ = 2,40 V, $p_o$ = 0,120 ; Δn = 0,139.
Für die Mischung ohne trans-4-(4-Pentenyl)cyclohexancarbonsäure-m-fluor -p-cyanophenylester ist $V_{10}$ = 2,80 V.

Mischung 14

11,58 Gew.-% p-(5-Butyl-2-pyrimidinyl)benzonitril,
3,50 Gew.-% p-(5-Pentyl-2-pyrimidinyl)benzonitril,
6,82 Gew.-% p-(5-Heptyl-2-pyrimidinyl)benzonitril,
14,86 Gew.-% trans-4-Butylcyclohexancarbonsäure-p-äthoxyphenylester,
13,80 Gew.-% trans-4-Pentylcyclohexancarbonsäure-p-methoxyphenylester,
17,99 Gew.-% trans-4-Butylcyclohexancarbonsäure-trans-4-butylcyclohexylester,
17,67 Gew.-% p-[trans-4-(4-Pentenyl)cyclohexyl]benzonitril,
13,78 Gew.-% 4'-[trans-4-(4-Pentenyl)cyclohexyl]-4-biphenylcarbonitril ;
Smp. < — 25 °C, Klp. 54 °C, nematisch ; $V_{10}$ = 1,259, $p_o$ = 0,119, $k_{33}/k_{11}$ = 1,14 ; Δn = 0,112.

Mischung 15

9,54 Gew.-% p-[trans-4-(3-Butenyl)cyclohexyl]benzonitril,
5,05 Gew.-% p-[trans-4-(3E-Pentenyl)cyclohexyl]benzonitril,
12,92 Gew.-% p-(trans-4-Propylcyclohexyl)phenylisothiocyanat,
4,94 Gew.-% 4-Aethoxy-1-(trans-4-propylcyclohexyl)äthyl]benzol,
4,16 Gew.-% 4-Aethyl-1-[2-(trans-4-propylcyclohexyl)äthyl]benzol,
16,58 Gew.-% 4-Aethoxy-1[2-(trans-4-pentylcyclohexyl)äthyl]benzol,
3,14 Gew.-% 4'-[trans-4-(3-Butenyl)cyclohexyl]-4-biphenylcarbonitril,
1,64 Gew.-% 4'-[trans-4-(3E-Pentenyl)cyclohexyl]-4-biphenylcarbonitril,
6,62 Gew.-% 4'-Aethyl-4-[trans-4-(3E-pentenyl)cyclohexyl]biphenyl,
8,63 Gew.-% 4'-Propyl-4-[trans-4-(3E-pentenyl)cyclohexyl]biphenyl,
9,88 Gew.-% 1-[2-(trans-4-Butylcyclohexyl)äthyl]-4-(trans-4-pentylcyclohexyl)benzol,
4,94 Gew.-% 4'-[2-(trans-4-Butylcyclohexyl)äthyl]-4-(trans-4-pentylcyclohexyl)biphenyl,
5,98 Gew.-% 4'-[2-(trans-4-Butylcyclohexyl)äthyl]-4-(trans-4-pentylcyclohexyl)-1,1'-äthylendibenzol,
5,98 Gew.-% 4-Aethyl-1-[trans-4-(4-pentenyl)cyclohexyl]benzol ;
Smp. < — 30 °C, Klp. 86 °C, nematisch ; $V_{10}$ = 2,803 V, $p_o$ = 0,118 ; $t_{on}$ (22 °C) = 27 ms, $t_{off}$ (22 °C) = 35 ms, $t_{on}$ (— 20 °C) = 345 ms, $t_{off}$ (— 20 °C) = 386 ms.

Mischung 16

15,22 Gew.-% p-(trans-4-Pentylcyclohexyl)benzonitril,
2,70 Gew.-% 4-Aethoxy-1-(trans-4-propylcyclohexyl)benzol,
7,71 Gew.-% p-(5-Butyl-2-pyrimidinyl)benzonitril,
4,54 Gew.-% p-(5-Heptyl-2-pyrimidinyl)benzonitril,
6,30 Gew.-% 4-Aethyl-1-[2-(trans-4-propylcyclohexyl)äthyl]benzol,
9,06 Gew.-% trans-4-Butylcyclohexancarbonsäure-p-äthoxyphenylester,
8,23 Gew.-% trans-4-Pentylcyclohexancarbonsäure-p-äthoxyphenylester,
13,50 Gew.-% trans-4-Pentylcyclohexancarbonsäure-p-propyloxyphenylester,
5,39 Gew.-% 4'-(trans-4-Pentylcyclohexyl)-4-biphenylcarbonitril,
5,53 Gew.-% p-[5-(trans-4-Aethylcyclohexyl)-2-pyrimidinyl]benzonitril,
11,82 Gew.-% 1-[2-(trans-4-Butylcyclohexyl)äthyl]-4-(trans-4-pentylcyclohexyl)benzol,
10,00 Gew.-% trans-5-(4-Pentenyl)-2-(p-butyloxyphenyl)-m-dioxan ;
Smp. < — 20 °C, Klp. 67 °C, nematisch ; $V_{10}$ = 1,616 V, $p_o$ = 0,126, $k_{33}/k_{11}$ = 1,07.
Für die entsprechende Mischung ohne trans-5-(4-Pentenyl)-2-(p-butyloxyphenyl)m-dioxan ist $V_{10}$ = 1,71 V, $p_o$ = 0,128 und $k_{33}/k_{11}$ = 1,19.

Mischung 17

7 Gew.-% 4'-Methyl-4-pentylbiphenyl,
10 Gew.-% p-(5-Butyl-2-pyrimidinyl)benzonitril,
12 Gew.-% trans-4-Butylcyclohexancarbonsäure-p-äthoxyphenylester,
11 Gew.-% trans-4-Pentylcyclohexancarbonsäure-p-methoxyphenylester,
17 Gew.-% trans-4-Pentylcyclohexancarbonsäure-trans-4-propylcyclohexylester,
3 Gew.-% 5-(p-Butylphenyl)-2-(p-pentylphenyl)pyrimidin,
5 Gew.-% 5-(trans-4-Aethylcyclohexyl)-2-(p-pentylphenyl)pyrimidin,
12 Gew.-% p-[trans-4-(4-Pentenyl)cyclohexyl]benzonitril,
12 Gew.-% trans-4-(4-Pentenyl)cyclohexancarbonsäure-p-äthoxyphenylester,
11 Gew.-% 4'-[trans-4-(4-Pentenyl)cyclohexyl]-4-biphenylcarbonitril ;

Smp. < — 20 °C, Klp. 60 °C, nematisch ; $V_{10}$ = 1,641 V, $p_0$ = 0,112.

Die Herstellung der Verbindungen der Formel I wird durch die folgenden Beispiele weiter veranschaulicht. C bedeutet eine kristalline, S eine smektische, $S_A$ eine smektisch A, $S_B$ eine smektisch B, N eine nematische und I die isotrope Phase.

Beispiel 1

a) In einem Sulfierkolben mit Thermometer, mechanischem Rührer, Tropftrichter und Festsubstanzzugaberohr wurden unter Argonbegasung 10,4 g Triphenyl-methoxymethyl-phosphoniumchlorid in 60 ml t-Butylmethyläther aufgeschlämmt und bei — 10 °C innert 10 Minuten mit 3,6 g festem Kalium-t-butylat versetzt. Nach beendeter Zugabe wurde noch während 30 Minuten bei — 10 °C gerührt und dann das tieforange, heterogene Reaktionsgemisch bei 0 °C tropfenweise mit einer Lösung von 4,2 g 4-(p-Cyanophenyl)cyclohexanon in 50 ml absolutem Tetrahydrofuran versetzt. Anschliessend wurde das Reaktionsgemisch noch 2 Stunden bei Raumtemperatur gerührt, dann auf 500 ml Hexan gegossen und filtriert. Niederdruckchromatographie (0,5 bar) des eingeengten Rückstandes (7,1 g) an Kieselgel mit Aethylacetat/Petroläter (Vol. 5 : 95) ergab 4,5 g (94 %) p-[4-(Methoxymethylen)cyclohexyl]benzonitril als farbloses Oel ; Reinheit 95 %, Rf-Wert (Aethylacetat/Petroläther Vol. 1 : 9) 0,30.

b) In einem Rundkolben wurde ein Gemisch von 4,2 g p-[4-(Methoxymethylen)cyclohexyl]benzonitril und 100 ml Tetrahydrofuran/2N Salzsäure (Vol. 4 : 1) während 30 Minuten zum Rückfluss erhitzt. Anschliessend wurde das Reaktionsgemisch auf 100 ml Wasser gegossen und dreimal mit je 100 ml Diäthyläther extrahiert. Die organischen Phasen wurden noch einmal mit 100 ml Wasser gewaschen, über Magnesiumsulfat getrocknet und eingeengt. Es resultierten 3,9 g (100 %) 4-(p-Cyanophenyl)cyclohexancarboxaldehyd als farbloses Oel, welches ohne weitere Reinigung in der nächsten Stufe eingesetzt wurde ; trans/cis-Verhältnis ca. 3 : 1 Rf-Wert (Aethylacetat/Petroläther Vol. 3 : 7) 0,41. Durch Kristallisation aus Hexan konnte reiner trans-4-(p-Cyanophenyl)cyclohexancarboxaldehyd erhalten werden ; Smp. 57,1 °C.

c) Eine Suspension von 29,0 g Methoxymethyl-triphenylphosphoniumchlorid in 200 ml t-Butylmethyläther wurde unter Argonbegasung bei — 10 °C innert 10 Minuten mit 9,7 g Kalium-t-butylat versetzt und noch 1 Stunde gerührt. Dann wurde das Gemisch bei —10 °C innert 15 Minuten tropfenweise mit einer Lösung von 12,0 g trans-4-(p-Cyanophenyl)cyclohexancarboxaldehyd in 90 ml t-Butylmethyläther versetzt und noch 1 Stunde bei 0 °C gerührt. Anschliessend wurde das Reaktionsgemisch auf 150 ml Wasser gegossen und dreimal mit je 150 ml Diäthyläther extrahiert. Die organischen Phasen wurden zweimal mit je 100 ml Wasser gewaschen, über Magnesiumsulfat getrocknet und eingeengt. Das resultierende, zähflüssige Oel wurde in 20 ml Aethylacetat gelöst und die klare Lösung mit 300 ml Petroläther verdünnt und 10 Minuten bei — 20 °C stehengelassen. Anschliessend wurde das ausgefallene Triphenylphosphinoxid abfiltriert und das Filtrat zur Trockene eingeengt. Niederdruckchromatographie (0,5 bar) des Rückstandes (16,3 g) an Kieselgel mit Aethylacetat/Petroläther (Vol. 5 : 95) ergab 10,1 g (74 %) p-[trans-4-(2-Methoxyvinyl)cyclohexyl]benzonitril als farblose Kristalle ; Rf-Wert (Aethylacetat/Petroläther Vol. 10 : 90) 0,32.

d) Eine Lösung von 10,1 g p-[trans-4-(2-Methoxyvinyl)cyclohexyl]benzonitril in 200 ml Tetrahydrofuran/2N Salzsäure (Vol. 4 : 1) wurde 1 Stunde zum Rückfluss erhitzt. Anschliessend wurde das Reaktionsgemisch auf 200 ml Wasser gegossen und dreimal mit je 150 ml Diäthyläther extrahiert. Die organischen Phasen wurden zweimal mit je 100 ml Wasser gewaschen, über Magnesiumsulfat getrocknet und eingeengt, wobei 9,8 g [trans-4-(p-Cyanophenyl)cyclohexyl]acetaldehyd als leicht gelbliche Kristalle (Reinheit 98,7 %) erhalten wurden. Umkristallisation einer Probe aus Hexan/t-Butylmethyläther (Vol. 1 : 1) ergab reinen Aldehyd mit Smp. 43,4 °C.

e) Eine Suspension von 4,5 g Methoxymethyl-triphenylphosphoniumchlorid in 40 ml t-Butylmethyläther wurde unter Argonbegasung bei — 10 °C innert 3 Minuten mit 1,5 g Kalium-t-butylat versetzt und noch 1 Stunde bei 0-5 °C gerührt. Dann wurde die Suspension bei 0 °C innert 5 Minuten tropfenweise mit einer Lösung von 2,0 g [trans-4-(p-Cyanophenyl)cyclohexyl]acetaldehyd in 20 ml t-Butylmethyläther versetzt und noch 2 Stunden bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Das resultierende, zähflüssige Oel wurde in 15 ml Aethylacetat gelöst und die klare Lösung mit 250 ml Petroläther versetzt. Nach 10 Minuten Stehenlassen bei — 20 °C wurde das ausgefallene Triphenylphosphinoxid abfiltriert und das Filtrat zur Trockene eingeengt. Niederdruckchromatographie (0,5 bar) des Rückstandes (3,3 g) an Kieselgel mit Aethylacetat/Petroläther (Vol. 5 : 95) ergab 2,04 g (91 %) p-[trans-4-(3-Methoxy-2-propenyl)cyclohexyl]benzonitril als farbloses Oel ; Rf-Wert (Aethylacetat/Petroläther Vol. 5 : 95) ergab 2,04 g (91 %) p-[trans-4-(3-Methoxy-2-propenyl)cyclohexyl]benzonitril als farbloses Oel ; Rf-Wert (Aethylacetat/Petroläther Vol. 10 : 90) 0,33.

f) Eine Lösung von 2,04 g p-[trans-4-(3-Methoxy-2-propenyl)cyclohexyl]benzonitril in 100 ml Tetrahydrofuran/2N Salzsäure (Vol. 4 : 1) wurde 1 Stunde zum Rückfluss erhitzt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt, wobei 1,9 g (99 %) 3-[trans-4-(p-Cyanophenyl)cylcohexyl]propionaldehyd als farblose Kristalle erhalten werden ; Rf-Wert (Aethylacetat/Petroläther Vol. 10 : 90) 0,15.

g) Eine Suspension von 6,4 g Methoxymethyl-triphenylphosphoniumchlorid in 80 ml t-Butylmethyläther wurde unter Argonbegasung bei 0 °C innert 3 Minuten mit 2,1 g Kalium-t-butylat versetzt und noch 1 Stunde bei 0-5 °C gerührt. Dann wurde das Gemisch bei 0 °C innert 5 Minuten tropfenweise mit einer Lösung von 3,0 g 3-[trans-4-(p-Cyanophenyl)cyclohexyl]propionaldehyd in 20 ml t-Butylmethyläther versetzt und noch 2 Stunden bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Der Rückstand wurde in 15 ml Aethylacetat gelöst und die klare Lösung mit 250 ml Petroläther versetzt. Nach 10 Minuten Stehenlassen bei — 20 °C wurde das ausgefallene Triphenylphosphinoxid abfiltriert und das Filtrat zur Trockene eingeengt. Niederdruckchromatographie (0,5 bar) des Rückstandes (5,5 g) an Kieselgel mit Aethylacetat/Petroläther (Vol. 5 : 95) ergab 2,95 g (88 %) p-[trans-4-(4-Methoxy-3-butenyl)cyclohexyl]benzonitril als farblose Kristalle.

h) Eine Lösung von 2,95 g p-[trans-4-(4-Methoxy-3-butenyl)cyclohexyl]benzonitril in 100 ml Tetrahydrofuran/2N Salzsäure (Vol. 4 : 1) wurde 15 Minuten zum Rückfluss erhitzt. Anschliessend wurde das Reaktionsgemisch auf 100 ml Wasser gegossen und dreimal mit je 100 ml Diäthylather extrahiert. Die organischen Phasen wurden zweimal mit je 100 ml Wasser gewaschen, über Magnesiumsulfat getrocknet und eingeengt, wobei 2,6 g (93 %) 4-[trans-4-(p-Cyanophenyl)cyclohexyl]butyraldehyd als leicht gelbliches Oel erhalten wurde ; Rf-Wert (Toluol/Aethylacetat Vol. 95 : 5) 0,23.

i) Eine Suspension von 7,34 g Methyl-triphenylphosphoniumbromid in 90 ml t-Butylmethyläther wurde unter Argonbegasung bei 0 °C innert 2 Minuten mit 2,3 g Kalium-t-butylat versetzt und noch 1 Stunde bei Raumtemperatur gerührt. Danach wurde das Gemisch innert 5 Minuten tropfenweise mit einer Lösung von 3,5 g 4-[trans-4-(p-Cyanophenyl)cyclohexyl]butylraldehyd in 20 ml t-Butylmethyläther versetzt und noch 20 Stunden bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Der Rückstand wurde in 15 ml Aethylacetat gelöst und die klare Lösung mit 250 ml Petroläther versetzt. Nach 10 Minuten Stehenlassen bei — 20 °C wurde das ausgefallene Triphenylphosphinoxid abfiltriert und das Filtrat zur Trockene eingeengt. Niederdruckchromatographie (0,5 bar) des öligen Rückstandes (4,8 g) an Kieselgel mit Aethylacetat/Petroläther (Vol. 5 : 95) ergab 3,1 g (89 %) gelbliches Oel. Umkristallisation bei — 20 °C aus Methanol ergab schliesslich 2,76 g (80 %) p-[trans-4-(4-Pentenyl)cyclohexyl]benzonitril als farblose Kristalle ; Smp. (C-I) 29,8 °C, Klp. (N-I) 10,2 °C.

In analoger Weise können folgende Verbindungen hergestellt werden :

4'-(trans-4-Allylcyclohexyl)-4-biphenylcarbonitril ; Smp. (C-N) 107,7 °C, Klp. (N-I) 181,7 °C,

4'-[trans-4-(4-Pentenyl)cyclohexyl]-4-biphenylcarbonitril ; Smp. (C-N) 77,4 °C, Klp. (N-I) 200,8 °C.

Beispiel 2

a) Eine Suspension von 5,1 g Propyl-triphenylphosphoniumbromid in 80 ml t-Butylmethyläther wurde unter Argonbegasung bei — 10 °C innert 5 Minuten mit 1,48 g Kalium-t-butylat versetzt und noch 60 Minuten bei Raumtemperatur gerührt. Danach wurde das Gemisch bei 0 °C innert 5 Minuten mit einer Lösung von 2,0 g [trans-4-(p-Cyanophenyl)cyclohexyl]acetaldehyd in 25 ml t-Butylmethyläther versetzt und noch 45 Minuten bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Der Rückstand wurde in 20 ml Aethylacetat gelöst und mit 250 ml Petroläther versetzt. Nach 10 Minuten Stehenlassen bei — 20 °C wurde das ausgefallene Triphenylphosphinoxid abfiltriert und das Filtrat zur Trockene eingeengt. Niederdruckchromatographie (0,5 bar) des gelblichen, öligen Rückstandes (3,33 g) an Kieselgel mit Aethylacetat/Petroläther (Vol. 3 : 97) ergab 2,1 g p-[trans-4-(2-Pentenyl)cyclohexyl]benzonitril als farbloses Oel, enthaltend 92,7 % p-[trans-4-(2Z-Pentenyl)cyclohexyl]benzonitril und 6,6 % p-[trans-4-(2E-Pentenyl)cyclohexyl]benzonitril. Dieses Material wurde ohne zusätzliche Reinigung weiter umgesetzt. Gewünschtenfalls kann aber dieses Isomerengemisch durch Chromatographie an mit Silbernitrat beschichtetem Kieselgel getrennt werden (wie in Absatz d illustriert).

b) Eine Lösung von 1,59 g 90-proz. m-Chlorperbenzoesäure in 60 ml Methylenchlorid wurde mit 4,0 g gemahlenem Kaliumcarbonat versetzt. Die erhaltene Suspension wurde bei 0 °C innert 5 Minuten tropfenweise mit einer Lösung von 2,1 g p-[trans-4-(2-Pentenyl)cyclohexyl]benzonitril (enthaltend 92,7 '

2Z-Isomer und 6,6 % 2E-Isomer) in 20 ml Methylenchlorid versetzt und dann noch 3 Stunden bei Raumtemperatur gerührt, wobei nach 1 Stunde und nach 2 Stunden nochmals je 0,8 g m-Chlorperbenzoesäure zugegeben wurden. Anschliessend wurde das Reaktionsgemisch auf 150 ml 10-proz. Natriumthiosulfat-Lösung gegossen und dreimal mit je 100 ml Methylenchlorid extrahiert. Die organischen Phasen wurden mit 100 ml 10-proz. Natriumthiosulfat-Lösung und mit 100 ml gesättigter Natriumcarbonat-Lösung gewaschen, über Magnesiumsulfat getrocknet und eingeengt. Dies ergab 2,2 g (99 %) p-[trans-4-(2,3-Epoxypentyl)cyclohexyl]benzonitril als farblose Kristalle ; Rf-Wert (Aethylacetat/Petroläther Vol. 10 : 90) 0,15. Dieses Material wurde ohne zusätzliche Reinigung weiterverwendet.

c) Eine Lösung von 2,6 g Triphenylphosphin in 30 ml Methylenchlorid wurde bei 0 °C tropfenweise mit einer Lösung von 0,519 ml Brom in 20 ml Methylenchlorid versetzt, bis eine leichte Gelbfärbung bestehen blieb. Die erhaltene gelbe Suspension wurde am Rotationsverdampfer vorsichtig zur Trockene eingeengt und der gelbliche, kristalline Rückstand am Hochvakuum (0,5 Torr) bei Raumtemperatur 1 Stunde getrocknet. Das erhaltene Triphenylphosphin-Brom wurde in 50 ml Toluol aufgeschlämmt und am Rotationsverdampfer nochmals zur Trockene eingeengt. Danach wurde der Rückstand in 30 ml Toluol aufgeschlämmt, mit einer Lösung von 2,2 g p-[trans-4-(2,3-Epoxypentyl)cyclohexyl]benzonitril in 10 ml Toluol versetzt und das Gemisch 2 Stunden bei 80 °C Badtemperatur gerührt. Niederdruckchromatographie (0,5 bar) des abgekühlten Reaktionsgemisches an Kieselgel mit Toluol ergab 2,83 g (84 %) p-[trans-4-(2,3-Dibrompentyl)cyclohexyl]benzonitril als gelbliches Oel (enthaltend 66,6 % erythro-Form und 32,8 % threo-Form) ; Rf-Wert (Aethylacetat/Petroläther Vol. 10 : 90) 0,33. Dieses Material wurde ohne zusätzliche Reinigung weiterverwendet.

d) Eine Lösung von 1,98 g p-[trans-4-(2,3-Dibrompentyl)cyclohexyl]benzonitril (erythro/threo 66,6 : 32,8) in 30 ml Eisessig wurde bei Raumtemperatur unter Argonbegasung mit 2,0 g Zinkpulver versetzt und die Suspension 1 Stunde bei Raumtemperatur gerührt. Anschliessend wurde das Reaktionsgemisch auf 100 ml Wasser gegossen und dreimal mit je 100 ml Petroläther extrahiert. Die organischen Phasen wurden zweimal mit je 100 ml Wasser gewaschen, über Magnesiumsulfat getrocknet und eingeengt. Niederdruckchromatographie (0,5 bar) des gelblichen Rückstandes (1,16 g) an mit Silbernitrat belegtem Kieselgel mit Hexan/Diäthyläther (Vol. 90 : 10) ergab 586 mg (48 %) p-[trans-4-(2E-Pentenyl)cyclohexyl]benzonitril und 171 mg (14 %) p-[trans-4-(2Z-Pentenyl)cyclohexyl]benzonitril als farblose Oele. Das 2E-Isomer hat einen Schmelzpunkt von 9,6 °C bzw. 16,1 °C (2 Modifikationen) und einen virtuellen Klärpunkt von — 67 °C ; das 2Z-Isomer hat einen Schmelzpunkt von — 7,7 °C und einen virtuellen Klärpunkt von — 54 °C.

Die Beschichtung von Kieselgel bzw. Dünnschichtplatten mit Silbernitrat wurde wie folgt durchgeführt :

34 g Silbernitrat wurde in 1 000 ml Acetonitril gelöst. Die Dünnschichtplatten wurden einmal kurz in die Silbernitrat-Lösung eingetaucht und danach 2 Stunden bei 80 °C im Vakuum (12 Torr) getrocknet. Dann wurden 300 g Kieselgel in die restliche Silbernitrat-Lösung gegeben, gut vermengt, am Rotationsverdampfer vorsichtig zur Trockene eingeengt und bei Raumtemperatur am Hochvakuum (0,5 Torr) 2 Stunden getrocknet.

## Beispiel 3

a) 10,0 g 4′-Brom-4-biphenylcarboxaldehyd und 5,31 g Kupfer-(I)-cyanid wurden unter Argonbegasung in 80 ml Dimethylformamid gelöst und die Lösung bei 180 °C Badtemperatur 15 Stunden zum Rückfluss erhitzt. Danach wurde das Reaktionsgemisch vorsichtig auf 200 ml 25-proz. Ammoniak gegossen und dreimal mit je 200 ml Methylenchlorid extrahiert. Die organischen Phasen wurden einmal mit 200 ml 25-proz. Ammoniak und zweimal mit je 200 ml Wasser gewaschen, über Magnesiumsulfat getrocknet und eingeengt. Niederdruckchromatographie (0,5 bar) des gelblichen, kristallinen Rückstandes (8,7 g) an Kieselgel ergab 5,85 g (74 %) 4′-Cyano-4-biphenylcarboxaldehyd als leicht gelbliche Kristalle ; Rf-Wert (Toluol/Aethylacetat Vol. 90 :10) 0,27.

b) Eine Suspension von 12,4 g Methoxymethyl-triphenylphosphoniumchlorid in 120 ml t-Butylmethyläther wurde unter Argonbegasung bei 0 °C innert 3 Minuten mit 4,1 g Kalium-t-butylat versetzt und noch 1 Stunde bei 0 °C gerührt. Danach wurde das Gemisch innert 10 Minuten mit einer Lösung von 5,0 g 4′-Cyano-4-biphenylcarboxaldehyd in 40 ml Tetrahydrofuran versetzt und noch 1,5 Stunden bei Raumtemperatur gerührt. Anschliessend wurde das Reaktionsgemisch auf 200 ml Wasser gegossen und dreimal mit je 150 ml Diäthyläther extrahiert. Die organischen Phasen wurden zweimal mit je 150 ml Wasser gewaschen, über Magnesiumsulfat getrocknet und eingeengt. Der erhaltene Rückstand wurde in 25 ml Aethylacetat gelöst und mit 350 ml Petroläther versetzt. Nach 10 Minuten Stehenlassen bei — 20 °C wurde das ausgefallene Triphenylphosphinoxid abfiltriert und das Filtrat zur Trockene eingeengt. Niederdruckchromatographie (0,5 bar) des dunkelbraunen Oels (9,5 g) an Kieselgel mit Aethylacetat/Petroläther (Vol. 10 : 90) ergab 5,2 g (91 %) 4′-(2-Methoxyvinyl)-4-biphenylcarbonitril als gelbliche Kristalle ; Rf-Wert (Aethylacetat/Petroläther Vol. 10 : 90) 0,17.

c) Ein Gemisch von 4,9 g 4'-(2-Methoxyvinyl)-4-biphenylcarbonitril und 80 ml Eisessig/Wasser (Vol. 2 : 1) wurde bei 100 °C Badtemperatur 1,5 Stunden zum Rückfluss erhitzt. Anschliessend wurde das Reaktionsgemisch auf 150 ml Wasser und mit 100 ml gesättigter Natriumhydrogencarbonat-Lösung gewaschen, über Magnesiumsulfat getrocknet und eingeengt. Es resultierten 4,8 g (4'-Cyano-4-biphenylyl)acetaldehyd als gelblicher, kristalliner Rückstand, welcher ohne zusätzliche Reinigung weiterverwendet wurde ; Rf-Wert (Toluol/Aethylacetat Vol. 90 : 10) 0,37.

d) (4'-Cyano-4-biphenylyl)acetaldehyd wurde in analoger Weise zu Absatz b) zu 4'-(3-Methoxy-2-propenyl)-4-biphenylcarbonitril umgesetzt ; Rf-Wert (Aethylacetat/Petroläther Vol. 10 : 90) 0,20.

e) 4'-(3-Methoxy-2-propenyl)-4-biphenylcarbonitril wurde in analoger Weise zu Absatz c) zum 3-(4'-Cyano-4-biphenylyl)propionaldehyd umgesetzt ; Rf-Wert (Toluol/Aethylacetat Vol. 95 : 5) 0,34.

Beispiel 4

a) In einem Sulfierkolben wurden unter Rühren und Stickstoffbegasung 149 g Methoxymethyl-triphenylphosphoniumchlorid und 860 ml t-Butylmethyläther bei Raumtemperatur vorgelegt, die Suspension auf — 10 °C gekühlt und innert 10 Minuten mit 51,6 g Kalium-t-butylat versetzt. Die Suspension wurde noch 30 Minuten bei — 10 °C bis 0 °C gerührt und dann innert 45 Minuten bei 0 °C tropfenweise mit einer Lösung von 47,3 g 4,4-Aethylendioxycyclohexanon in 720 ml Tetrahydrofuran versetzt. Die orange Suspension wurde noch 2 Stunden bei Raumtemperatur weiter gerührt, dann auf 5 l Hexan gegossen, 10 Minuten gerührt und genutscht. Das Filtrat wurde im Vakuum eingeengt und das erhaltene gelbbräunliche Oel (104,1 g) mit 500 ml Hexan versetzt und genutscht. Das Filtrat wurde im Vakuum eingeengt, wobei 61,7 g gelbbräunliches Oel erhalten wurden. Chromatographische Trennung dieses Rohproduktes an Kieselgel mit Methylenchlorid/Aceton (Vol. 98 : 2 und 95 : 5) ergab schliesslich 53,5 g 1,1-Aethylendioxy-4-(methoxymethylen)cyclohexan als farbloses Oel.

b) In einem Rundkolben wurde unter Stickstoffbegasung ein Gemisch von 28,2 g 1,1-Aethylendioxy-4-(methoxymethylen-cyclohexan, 770 ml Eisessig und 385 ml Wasser 1 Stunde zum Rückfluss erhitzt. Danach wurde die gelbliche klare Lösung auf Raumtemperatur gekühlt, mit 800 ml Wasser verdünnt und dreimal mit je 700 ml Methylenchlorid extrahiert. Die organischen Phasen wurden zweimal mit je 500 ml 10 %-iger (Gew./Vol.) Natriumcarbonat-Lösung gewaschen, über Natriumsulfat getrocknet, filtriert und eingeengt. Chromatographische Trennung der erhaltenen bräunlichen Flüssigkeit (18,5 g) an Kieselgel mit Methylenchlorid als Eluens ergab schliesslich 16,7 g 4-Formylcyclohexanon als bräunliche Flüssigkeit.

c) In einem Sulfierkolben wurden unter Rühren und Stickstoffbegasung 63,3 g p-Cyanobenzyltriphenylphosphoniumchlorid, 17,2 g Kalium-t-butylat und 195 ml Aethylenglykoldimethyläther vorgelegt, wobei die Innentemperatur bis auf 44 °C anstieg. Die braune Suspension wurde auf 0 °C gekühlt und innert 2 Minuten mit einer Lösung von 16,7 g 4-Formylcyclohexanon in 100 ml Aethylenglykoldimethyläther versetzt. Danach wurde das Kühlbad entfernt und das Reaktionsgemisch noch 3,5 Stunden bei Raumtemperatur gerührt. Anschliessend wurde die Suspension auf 500 ml Wasser gegossen und dreimal mit je 600 ml Methylenchlorid extrahiert. Die organischen Phasen wurden zweimal mit je 500 ml 10 %-iger (Gew./Vol.) Kochsalzlösung gewaschen, über Natriumsulfat getrocknet, filtriert und eingeengt, wobei 76,9 g einer bräunlichen Paste zurückblieben. Chromatographische Trennung dieses Rohproduktes an Kieselgel mit Methylenchlorid als Eluens ergab 33,0 g 4-[2-(p-Cyanophenyl)vinyl]cyclohexanon als gelbbräunliches Oel.

d) In einem Rundkolben mit Magnetrührer wurde ein Gemisch von 33,0 g 4-[2-(p-Cyanophenyl)vinyl]-cyclohexanon, 520 ml Toluol, 260 ml Aethanol und 3,2 Palladium/Kohle (5 %) bei Raumtemperatur vorgelegt und das Gemisch bis zum Stillstand der Wasserstoffaufnahme hydriert. Anschliessend wurde die schwarze Suspension genutscht (Nachwaschen mit Toluol) und das Filtrat im Vakuum eingeengt. Das erhaltene, leicht trübe, gelbliche Oel (34,1 g) wurde an Kieselgel chromatographisch getrennt. Methylenchlorid/Hexan (Vol. 1 : 1), Methylenchlorid/Hexan (Vol. 8 : 2) und Methylenchlorid eluierten 25,6 g gelbliches Oel, welches aus t-Butylmethyläther kristallisiert wurde. Hierbei wurden 22,6 g 4-[2-(p-Cyanophenyl)äthyl]cyclohexanon als farblose Kristalle mit Smp. 62,5-64,3 °C erhalten.

e) Eine Suspension von 54,4 g Methoxymethyl-triphenylphosphoniumchlorid in 315 ml t-Butylmethyläther wurde unter Stickstoffbegasung bei — 10 °C mit 18,8 g Kalium-t-butylat versetzt und die orange Suspension noch 30 Minuten bei 0 °C gerührt. Anschliessend wurde das Gemisch bei 0 °C innert 30 Minuten tropfenweise mit einer Lösung von 25,1 g 4-([2-(p-Cyanophenyl)äthyl]cyclohexanon in 260 ml Tetrahydrofuran versetzt und das Gemisch noch 3 Stunden bei Raumtemperatur gerührt. Danach wurde das Reaktionsgemisch auf 2,6 l Hexan gegossen, 10 Minuten gerührt und genutscht (Nachwaschen mit Hexan). Das Filtrat wurde im Vakuum bei 50 °C eingeengt, wobei 47,7 g gelber, teilweise kristalliner Rückstand erhalten wurden. Niederdruckchromatographie (0,3 bar) des Rückstandes an Kieselgel mit

Hexan/Methylenchlorid (Vol. 4 : 1) und Methylenchlorid ergab 21,0 g p-[2-(4-Methoxymethylencyclohexyl)äthyl]benzonitril als gelbliches Oel ; Rf-Wert (Methylenchlorid) 0,43.

f) Ein Gemisch von 22,5 g p[2-(4-Methoxymethylencyclohexyl)äthyl]benzonitril und 300 ml Tetrahydrofuran/2N Salzsäure (Vol. 4 : 1) wurde unter Stickstoffbegasung 30 Minuten am Rückfluss gekocht. Das abgekühlte Reaktionsgemisch wurde auf 450 ml Wasser gegossen und dreimal mit je 300 ml Diäthyläther extrahiert. Die organischen Phasen wurden mit 200 ml Wasser gewaschen, über Natriumsulfat getrocknet und im Vakuum bei 50 °C eingeengt. Es resultierten 21,9 g 4-[2-(p-Cyanophenyl)äthyl]cyclohexancarboxaldehyd (trans/cis = 4 : 1) als farbloses Oel ; Rf-Wert (Methylenchlorid) 0,3.

g) Ein Gemisch von 21,9 g 4-[2-(p-Cyanophenyl)äthyl]cyclohexancarboxaldehyd (trans/cis = 4 : 1) und 167 ml 0,1N methanolischer Kaliumhydroxid-Lösung wurde unter Rühren und Stickstoffbegasung auf 0 °C gekühlt, innert 20 Minuten portionenweise mit 3,5 g Natriumborhydrid versetzt und danach noch 30 Minuten bei 0 °C weitergerührt. Anschliessend wurde die graue Suspension vorsichtig auf 420 ml Eiswasser gegossen und dreimal mit je 300 ml Methylenchlorid extrahiert. Die organischen Phasen wurden zweimal mit je 150 ml Wasser gewaschen, über Natriumsulfat getrocknet und im Vakuum bei 50 °C eingeengt. Der farblose, kristalline Rückstand von p-[2-(4-Hydroxymethylcyclohexyl)äthyl]benzonitril (22,0 g, trans/cis = 89,4 : 10,6) wurde in 100 ml Aethylacetat warm gelöst, mit 100 ml Hexan versetzt und unter Rühren auf 15 °C gekühlt, wobei Kristallisation eintrat. Dann wurde das Gemisch unter Rühren mit weiteren 300 ml Hexan versetzt, genutscht (Nachwaschen mit Hexan) und der Niederschlag im Vakuum bei 50 °C getrocknet. Die Mutterlauge wurde in analoger Weise aufgearbeitet und dann die beiden Kristallisate (insgesamt 17,3 g) nochmals aus Aethylacetat/Hexan umkristallisiert. Es resultierten 16,2 g (74 %) reines p-[2-(trans-4-Hydroxymethylcyclohexyl)äthyl]benzonitril als farblose Kristalle mit Smp. 119,2-121-1 °C.

h) Ein Gemisch von 9,3 g Oxalylchlorid und 166 ml Methylenchlorid wurde unter Rühren und Stickstoffbegasung bei —60 °C innert 10 Minuten tropfenweise mit einer Lösung von 11,4 g Dimethylsulfoxid in 33 ml Methylenchlorid versetzt. Danach wurde das Gemisch bei —60 °C innert 15 Minuten tropfenweise mit einer Lösung von 16,2 g p-[2-(trans-4-Hydroxymethylcyclohexyl)äthyl]benzonitril in 66 ml Methylenchlorid versetzt und noch 15 Minuten bei —60 °C gerührt. Anschliessend wurde das Reaktionsgemisch bei —60 °C innert 15 Minuten tropfenweise mit 46,6 ml Triäthylamin versetzt, dann innert 30 Minuten auf Raumtemperatur erwärmt, auf 330 ml Wasser gegossen und dreimal mit je 200 ml Methylenchlorid extrahiert. Die organischen Phasen wurden zweimal mit je 200 ml Wasser gewaschen, über Natriumsulfat getrocknet und im Vakuum bei 50 °C eingeengt. Niederdruckchromatographie (0,3 bar) der erhaltenen, bräunlichen Kristalle (16,5 g) an Kieselgel mit Methylenchlorid ergab 14,4 g (89,6 %) reinen trans-4-[2-(p-Cyanophenyl)äthyl]cyclohexancarboxaldehyd als farblose Kristalle mit Smp. 90,0-91,2 °C.

i) In analoger Weise zu Beispiel 1 c)-i) wurde trans-4-[2-(p-Cyanophenyl)äthyl]cyclohexancarboxaldehyd zu p-[2-(trans-4-(4-Pentenyl)cyclohexyl)äthyl]benzonitril umgesetzt ; Smp. (C-I) 25,3 °C, Klp. (N-I) 17,2 °C.

In analoger Weise kann folgende Verbindung hergestellt werden :

p-[2-(trans-4-Allylcyclohexyl)äthyl]benzonitril ; Smp. (C-I) 39,7 °C, Klp. (N-I) —9,3 °C.

## Beispiel 5

Eine Suspension von 3,16 g Aethyl-triphenylphosphoniumbromid in 50 ml trockenem t-Butylmethyläther wurde unter Argonbegasung bei —10 °C mit 960 mg Kalium-t-butylat versetzt und noch 45 Minuten bei 0 °C gerührt. Danach wurde das Gemisch auf —20 °C abgekühlt, innert 5 Minuten mit einer Lösung von 1,45 g 4-[trans-4-(p-Cyanophenyl)cyclohexyl]-butylraldehyd (Reinheit 90 %) in 20 ml t-Butylmethyläther versetzt und noch 30 Minuten unter Erwärmen auf Raumtemperatur gerührt. Anschliessend wurde das Reaktionsgemisch auf 100 ml Wasser gegossen und dreimal mit je 100 ml Diäthyläther extrahiert. Die organischen Phasen wurden mit 100 ml Wasser gewaschen, über Magnesiumsulfat getrocknet und eingeengt. Niederdruckchromatographie (0,5 bar) des Rückstandes an Kieselgel mit Aethylacetat/Petroläther (Vol. 3 : 97) ergab p-[trans-4-(4-Hexenyl)cyclohexyl]benzonitril (4Z : 4E = 88 : 12) als farbloses Oel. Zusätzliche Niederdruckchromatographie (0,5 bar) dieses Materials an 140 g mit Silbernitrat beschichtetem Kieselgel (hergestellt gemäss Beispiel 2) mit Hexan/Diäthyläther (Vol. 3 : 1) lieferte 750 mg p-[trans-4-(4Z-Hexenyl)cyclohexyl]benzonitril. Zweimalige Umkristallisation aus Methanol ergab schliesslich 468 mg farblose Kristalle mit Smp. (C-I) 27,4 °C und Klp. (N-I) 4,5 °C.

In analoger Weise kann folgende Verbindung hergestellt werden :

4'-[trans-4-(2Z-Pentenyl)cyclohexyl]-4-biphenylcarbonitril ; Smp. (C-N) 81,9 °C, Klp. (N-I) 165,0 °C.

## Beispiel 6

a) Ein Gemisch von 9,88 g 4'-[trans-4-(4-Pentenyl)cyclohexyl]-4-biphenylcarbonitril, 735 ml Diäthylenglykol und 79,9 g Kaliumhydroxid wurde unter Rühren und Stickstoffbegasung auf 150-160 °C erhitzt und 1 Stunde bei dieser Temperatur gehalten. Anschliessend wurde das Reaktionsgemisch auf Raumtemperatur abgekühlt und unter Kühlung mit einem Eisbad mit 230 ml halbkonzentrierter Salzsäure versetzt. Danach wurde das Reaktionsgemisch auf 1,5 l Wasser gegossen, bei Raumtemperatur 10 Minuten gerührt und dann genutscht. Der Rückstand wurde mit Wasser neutral gewaschen und im Vakuum bei 60 °C getrocknet, wobei 10,3 g (98,5 %) 4'-[trans-4-(4-Pentenyl)cyclohexyl]-4-biphenylcarbonsäure als farblose Kristalle erhaltene wurden.

b) Ein Gemisch von 10,3 g 4'-[trans-4-(4-Pentenyl)cyclohexyl]-4-biphenylcarbonsäure und 300 ml Aceton wurde unter Rühren und Stickstoffbegasung auf 2 °C gekühlt, innert 10 Minuten tropfenweise mit einer Lösung von 8,2 ml Triäthylamin in 58 ml Aceton versetzt und noch 5 Minuten gerührt. Danach wurde das Gemisch bei 0-2 °C innert 10 Minuten tropfenweise mit einer Lösung von 7,1 ml Chlorameisensäureäthylester in 29 ml Aceton versetzt. Das Gemisch wurde noch 30 Minuten bei 0-2 °C gerührt und innert 10 Minuten tropfenweise mit einer Lösung von 5,25 g Natriumazid in 29 ml Wasser versetzt. Das Reaktionsgemisch wurde noch 2,25 Stunden unter Kühlung mit einem Eisbad weitergerührt, dann auf 1 l Wasser gegossen und einmal mit 1 l und einmal mit 500 ml Diäthyläther extrahiert. Die Aetherphasen wurden mit Wasser gewaschen, über Natriumsulfat getrocknet, filtriert und eingeengt. Der beige kristalline Rückstand (12,2 g) wurde an Kieselgel mit Methylenchlorid chromatographisch getrennt, wobei 9,9 g (89,5 %) 4'-[trans-4-(4-Pentenyl)cyclohexyl]-4-biphenylcarbonsäureazid als farblose Kristalle erhalten wurden.

c) Ein Gemisch von 9,9 g 4'-[trans-4-(4-Pentenyl)cyclohexyl]-4-biphenylcarbonsäureazid und 215 ml Propanol wurde unter Stickstoffbegasung 30 Minuten am Rückfluss gekocht. Danach wurde das Reaktionsgemisch eingeengt, wobei 10,7 g (99,5 %) Propyl-4'-[trans-4-(4-Pentenyl)cyclohexyl]-4-biphenylcarbamat als farblose Kristalle erhalten wurden.

d) Ein Gemisch von 10,7 g Propyl-4'-[trans-4-(4-Pentenyl)cyclohexyl]-4-biphenylcarbamat, 645 ml Diäthylenglykol, 90,1 g Kaliumhydroxid und 164 ml Wasser wurde 17,5 Stunden in einem Oelbad von 170 °C erhitzt, dann auf Raumtemperatur abgekühlt, auf 800 ml Wasser gegossen und dreimal mit Methylenchlorid extrahiert. Die organischen Phasen wurden zweimal mit Wasser gewaschen, über Natriumsulfat getrocknet, filtriert und eingeengt. Der bräunliche kristalline Rückstand (8,2 g) wurde an Kieselgel mit Methylenchlorid/Hexan (Vol. 7 : 3) chromatographisch gereinigt, wobei 8,1 g (96 %) 4-Amino-4'-[trans-4-(4-Pentenyl)cyclohexyl]-4-biphenyl als beige Kristalle erhalten wurden.

e) Eine Lösung von 8,1 g 4-Amino-4'-[trans-4-(4-Pentenyl)cyclohexyl]-4-biphenyl in 144 ml Chloroform wurde unter Rühren und Stickstoffbegasung auf 4 °C abgekühlt und dann mit 7,14 ml Triäthylamin versetzt. Anschliessende wurde das Gemisch bei 0-5 °C innert 20 Minuten tropfenweise mit einer Lösung von 2,33 ml Thiophosgen (Reinheit 95 %) in 71 ml Chloroform versetzt. Das Reaktionsgemisch wurde noch 30 Minuten bei Raumtemperatur weiter gerührt und dann 1,5 Stunden am Rückfluss gekocht. Anschliessend wurde das Reaktionsgemisch auf Raumtemperatur abgekühlt und mit 110 ml 3N Ammoniak-Lösung und mit 200 ml Wasser gewaschen. Die wässrigen Phasen wurden mit Methylenchlorid nachextrahiert. Die organischen Phasen wurden über Natriumsulfat getrocknet, filtriert und eingeengt. Zweimalige chromatographische Trennung des beigen, kristallinen Rückstandes (9,3 g) an Kieselgel zuerst mit Hexan/Methylenchlorid (Vol. 8 : 2) und dann mit Hexan/Chloroform (Vol. 8 : 2), ergab 8,2 g farbloses, kristallines Produkt. Umkristallisation aus Aceton ergab 6,788 g (74,1 %) 4'-[trans-4-(4-Pentenyl)cyclohexyl]-4-biphenylylisothiocyanat mit Smp. (C-$S_B$) 95,5 °C, $S_B$-$S_A$ 111,5 °C, $S_A$-N 113,6 °C, Klp. (N-I) 213,5 °C.

In analoger Weise können folgende Verbindungen hergestellt werden :

p-[trans-4-(4-Pentenyl)cyclohexyl]phenylisothiocyanat ; Smp. (C-I) 38,2 °C, virtueller Klp. — 10 °C,

p-[5-(4-Pentenyl)-2-pyrimidinyl]phenylisothiocyanat ; Smp. (C-$S_A$) 38,2 °C, Klp. ($S_A$-I) 62,5 °C.

Beispiel 7

a) Eine Lösung von Methylmagnesiumjodid in Diäthyläther (hergestellt aus 195 mg Magnesium-Späne und 0,495 ml Methyljodid in 15 ml Diäthyläther) wurde bei Raumtemperatur tropfenweise mit einer Lösung von 1,0 g p-[trans-4-(4-Pentenyl)cyclohexyl]benzonitril versetzt. Das Gemisch wurde 15 Minuten zum Rückfluss erhitzt. Anschliessend wurden 15 ml Toluol zum Reaktionsgemisch zugegeben, der Diäthyläther abdestilliert und das Gemisch nochmals 1,5 Stunden zum Rückfluss erhitzt. Danach wurde das Reaktionsgemisch bei 0 °C vorsichtig mit gesättigter Ammoniumchlorid-Lösung versetzt und dreimal in Diäthyläther/Wasser verteilt. Die organischen Extrakte wurden zweimal mit Wasser gewaschen, über Magnesiumsulfat getrocknet, filtriert und eingedampft. Chromatographische Trennung des gelben kristallinen Rückstandes (1,1 g) an Kieselgel mit Essigester/Petroläther (Vol. 5 : 95) ergab 0,96 g (90 %) p-[trans-4-(4-Pentenyl)cyclohexyl]acetophenon als leicht gelbe Kristalle ; Smp. 47,6 °C.

b) Eine Lösung von 0,9 g p-[trans-4-(4-Pentenyl)cyclohexyl]acetophenon in 8 ml Aethanol und 8 ml

Diäthylenglykol wurde unter Argonbegasung mit 350 ml Hydrazinhydrat versetzt und dann unter Rühren 1 Stunde zum Rückfluss erhitzt (Badtemperatur 110 °C). Anschliessend wurde das Gemisch mit 421 mg festem Kaliumhydroxid versetzt, die Badtemperatur auf 210 °C erhöht und der Aethanol abdestilliert. Nach 3 Stunden bei 210 °C wurde die Reaktion abgebrochen und das Gemisch dreimal in Wasser-/Petroläther verteilt. Die organischen Extrakte wurden zweimal mit Wasser gewaschen, über Magnesiumsulfat getrocknet und eingedampft. Chromatographische Trennung des Rückstandes (0,85 g) an Kieselgel mit Essigester/Petroläther (Vol. 3 : 97) ergab 0,76 g (90 %) 4-Aethyl-1-[trans-4-(4-Pentenyl)cyclohexyl]benzol als farbloses Oel ; Smp. (C-I) — 10,6 °C.

In analoger Weise wurden folgende Verbindungen hergestellt :

4'-Acetyl-4'-[trans-4-(4-Pentenyl)cyclohexyl]biphenyl ; nicht kristallisierbar bei Raumtemperatur,

4'-Aethyl-4'-[trans-4-(4-Pentenyl)cyclohexyl]biphenyl ; Klp. (S-I) 147,5 °C, nicht kristallisierbar bis 0 °C,

4'-Propionyl-4'-[trans-4-(4-Pentenyl)cyclohexyl]biphenyl,

4'-Propyl-4'-[trans-4-(4-Pentenyl)cyclohexyl]biphenyl ; Klp. (S-I) 153,3 °C, nicht kristallisierbar bis — 50 °C,

p-[2-(trans-4-Allylcyclohexyl)äthyl]acetophenon ; Smp. 33,9 °C,

4-Aethyl-1-[2-(trans-4-Allylcyclohexyl)äthyl]benzol ; nicht kristallisierbar bis unter — 20 °C, Klp. — 21 °C,

p-[2-(trans-4-(4-Pentenyl)cyclohexyl)äthyl]acetophenon ; Smp. 32,4 °C,

4-Aethyl-1-[2-(trans-4-(4-pentenyl)cyclohexyl)äthyl]benzol ; Smp. — 16,9 °C, Klp. — 5,1 °C.

## Beispiel 8

a) Eine Suspension von 11,5 g 3,3-Aethyldendioxypropyl-triphenylphosphoniumbromid in 90 ml t-Butylmethyläther wurde bei 0 °C mit 3,02 g Kalium-t-butylat versetzt und dann bei Raumtemperatur 40 Minuten gerührt. Danach wurde das Gemisch bei 5 °C tropfenweise mit einer Lösung von 2,6 g p-Cyanobenzaldehyd in 30 ml Tetrahydrofuran versetzt und noch 1 Stunde bei Raumtemperatur gerührt. Anschliessend wurde das Reaktionsgemisch mit Wasser versetzt. Die Phasen wurden getrennt und die wässrige Phase wurde zweimal mit Methylenchlorid nachextrahiert. Die organischen Phasen wurden zweimal mit Wasser gewaschen, über Magnesiumsulfat getrocknet und eingeengt. Der Rückstand wurde in heissem Essigester gelöst, die Lösung mit Petroläther versetzt und das ausgefallene Triphenylphosphinoxid abgenutscht. Das nach Einengen des Filtrates erhaltene gelbe Oel (4,5 g) wurde an Kieselgel mit Petroläther und Petroläther/Essigester chromatographisch gereinigt. Es resultierten 3,73 g (89 2 %) p-(4,4-Aethylendioxy-1-butenyl)benzonitril als leicht gelbliches Oel.

b) Eine Lösung von 3,7 g p-(4,4-Aethylendioxy-1-butenyl)benzonitril in 50 ml Toluol wurde mit 350 mg Palladium/Kohle (5 %) versetzt und 2,5 Stunden hydriert (Wasserstoffverbrauch 385 ml). Dann wurde das Reaktionsgemisch filtriert (Nachwaschen mit Diäthyläther) und das Filtrat eingedampft. Es resultierten 308 mg (82 %) p-(4,4-Aethylendioxybutyl)benzonitril als farbloses, teilweise kristallisierendes Oel.

c) Ein Gemisch von 2,8 g p-(4,4-Aethylendioxybutyl)benzonitril, 56 ml Tetrahydrofuran und 56 ml 10 %-iger Salzsäure wurde 3 Stunden bei Raumtemperatur gerührt und dann über Nacht stehengelassen. Danach wurde das Reaktionsgemisch mit Wasser verdünnt und dreimal mit Diäthyläther extrahiert. Die organischen Phasen wurden zweimal mit Wasser gewaschen, über Magnesiumsulfat getrocknet, filtriert und eingeengt. Es resultierten 2,6 g 4-(p-Cyanophenyl)butyraldehyd, welche ohne zusätzliche Reinigung weiterverwendet wurden.

d) Eine Suspension von 6,5 g Methyl-triphenylphosphoniumbromid in 50 ml t-Butylmethyläther wurde bei — 5 °C mit 2,1 g Kalium-t-butylat versetzt und dann bei Raumtemperatur 40 Minuten gerührt. Anschliessend wurde das Gemisch bei 0 °C mit einer Lösung von 2,6 g 4-(p-Cyanophenyl)butyraldehyd in 30 ml t-Butylmethyläther versetzt und 1 Stunde bei Raumtemperatur gerührt. Danach wurde das Reaktionsgemisch mit Wasser versetzt und dreimal mit Diäthyläther extrahiert. Die organischen Phasen wurden zweimal mit Wasser gewaschen, über Magnesiumsulfat getrocknet, filtriert und eingeengt. Der kristalline Rückstand wurde in heissem Essigester gelöst, die Lösung mit Petroläther versetzt und das ausgefallene Triphenylphosphin abfiltriert. Das nach Einengen des Filtrates erhaltene Oel wurde an Kieselgel mit Essigester/Petroläther (Vol. 5 : 95) chromatographisch gereinigt, wobei 1,92 g p-(4-Pentenyl)benzonitril als leicht gelbliche Flüssigkeit isoliert wurden.

e) Ein Gemisch von 1,9 g p-(4-Pentenyl)benzonitril und einer 10 %-igen Lösung von Kaliumhydroxid in Diäthylenglykol wurde 2 Stunden bei 180 °C gekocht. Dann wurde das Reaktionsgemisch auf Raumtemperatur abgekühlt, mit 23 %-iger Salzsäure auf pH 3 gestellt, mit Wasser verdünnt und viermal mit Methylenchlorid extrahiert. Die organischen Phasen wurden zweimal mit Wasser gewaschen, über Magnesiumsulfat getrocknet, filtriert und eingeengt, wobei 2,12 g p-(4-Pentenyl)benzoesäure als braune Kristalle isoliert wurden.

Beispiel 9

a) Eine Suspension von 829 mg Natriumborhydrid in 20 ml Methanol/Diäthyläther (Vol. 9 : 1) wurde bei 0 °C innert 5 Minuten tropfenweise mit einer Lösung von 3,0 g trans-4-Cyanocyclohexancarboxaldehyd in 30 ml Methanol/Diäthyläther (Vol. 9 : 1) versetzt. Das Reaktionsgemisch wurde noch 2 Stunden bei 10 °C gerührt, dann mit 10 ml verdünnter Salzsäure versetzt und in Methylenchlorid/Wasser verteilt. Die wässrige Phase wurde dreimal mit Methylenchlorid extrahiert. Die organischen Phasen wurden zweimal mit Wasser gewaschen, über Magnesiumsulfat getrocknet, filtriert und eingeengt. Es resultierten 3,0 g (98 %) trans-4-(Hydroxymethyl)cyclohexancarbonitril als farbloses Oel.

b) Eine Lösung von 3,0 g trans-4-(Hydroxymethyl)cyclohexancarbonitril in 10 ml Pyridin wurde bei 0 °C innert 3 Minuten tropfenweise mit einer Lösung von 6,81 g p-Tosylchlorid in 10 ml Pyridin versetzt. Das Reaktionsgemisch wurde 15 Stunden bei Raumtemperatur gerührt, dann mit 50 ml 25 %-iger Salzsäure sauer gestellt (pH ca. 2) und in Chloroform/Wasser verteilt. Die wässrige Phase wurde dreimal mit Chloroform extrahiert. Die organischen Phasen wurden zweimal mit Wasser gewaschen, über Magnesiumsulfat getrocknet, filtriert und eingeengt. Es resultierten 6,3 g (99 %) trans-4-(p-Tosyloxymethyl)cyclohexancarbonitril als farblose Kristalle.

c) Eine Lösung von 6,3 g trans-4-(p-Tosyloxymethyl)cyclohexancarbonitril in 80 ml Aceton wurde mit 3,87 g Natriumjodid versetzt und das Gemisch unter Rühren 15 Stunden zum Rückfluss erhitzt. Danach wurde die weisse Suspension filtriert und das Filtrat eingeengt. Der Rückstand wurde in Wasser-/Chloroform verteilt. Die wässrige Phase wurde dreimal mit Chloroform extrahiert. Die organischen Phasen wurden zweimal mit Wasser gewaschen, über Magnesiumsulfat getrocknet, filtriert und eingeengt. Es resultierten 4,8 g (89 %) trans-4-(Jodmethyl)cyclohexancarbonitril als gelbliches Oel.

d) Eine Suspension von 9,14 g Kupfer-(I)-jodid in 90 ml Tetrahydrofuran wurde bei —78 °C innert 5 Minuten mittels einer Spritze mit 25,7 ml einer 1,5M Lösung von Methyllithium in Tetrahydrofuran versetzt. Die Suspension wurde noch 45 Minuten bei —78 °C gerührt, dann auf 0 °C erwärmen gelassen und noch 3 Minuten bei 0 °C gerührt. Danach wurde die Suspension wieder auf —78 °C abgekühlt und innert 5 Minuten mittels einer Stahlkanüle mit einer aus 4,56 ml 4-Brom-1-buten und 1,1 g Magnesium in 60 ml Tetrahydrofuran hergestellten Grignardlösung versetzt. Die Suspension wurde noch 20 Minuten bei —78 °C gerührt, dann auf 15 °C erwärmen gelassen und noch 5 Minuten bei 15 °C gerührt. Danach wurde die Suspension wieder auf —78 °C abgekühlt und innert 5 Minuten tropfenweise mit einer Lösung von 4,8 g trans-4-(Jodmethyl)cyclohexancarbonitril in 30 ml Tetrahydrofuran versetzt. Danach wurde das Reaktionsgemisch erwärmen gelassen und noch 30 Minuten bei 16 °C gerührt. Anschliessend wurde das Reaktionsgemisch im Kühlbad vorsichtig mit ca. 50 ml Ammoniumchlorid-Lösung versetzt und in Methylenchlorid/Ammoniumchlorid-Lösung verteilt. Die wässrige Phase wurde dreimal mit Methylenchlorid extrahiert. Die organischen Phasen wurden einmal mit Ammoniumchlorid-Lösung und zweimal mit Wasser gewaschen, über Magnesiumsulfat getrocknet, filtriert und eingeengt. Chromatographische Trennung des gelben, öligen Rückstandes (3,4 g) an Kieselgel mit Essigester/Petroläther (Vol. 5 : 95) ergab 2,9 g (85 %) trans-4-(4-Pentenyl)cyclohexancarbonitril als leicht gelbes Oel.

e) Eine Lösung von 2,9 g trans-4-(4-Pentenyl)cyclohexancarbonitril in 80 ml Diäthylenglykol wurde mit 8,0 g Kaliumhydroxid versetzt und das Gemisch 3,5 Stunden bei einer Badtemperatur von 180 °C gerührt. Anschliessend wurde das Reaktionsgemisch mit Eis versetzt, mit 25 ml 25 %-iger Salzsäure sauer gestellt und in Methylenchlorid/Wasser verteilt. Die wässrige Phase wurde dreimal mit Methylenchlorid extrahiert. Dir organischen Phasen wurden zweimal mit Wasser gewaschen, über Magnesiumsulfat getrocknet, filtriert und eingeengt. Chromatographische Trennung des braunen, öligen Rückstandes (3,5 g) an Kieselgel mit Essigester/Petroläther (Vol. 10 : 90) ergab 2,9 g (90 %) trans-4-(4-Pentenyl)cyclohexancarbonsäure als gelbliches Oel.

Beispiel 10

a) Eine Lösung von 400 mg p-(3-Hydroxypropyl)phenol und 0,421 ml Triäthylamin in 2,6 ml Methylenchlorid wurde bei 0 °C portionenweise mit 539 mg p-Tosylchlorid versetzt und das Gemisch noch 5 Minuten bei 0 °C und 15 Minuten bei —5 °C gerührt. Anschliessend wurde das Reaktionsgemisch mit Wasser verdünnt, mit verdünnter Salzsäure leicht sauer gestellt und dreimal mit Methylenchlorid extrahiert. Die organischen Phasen wurden zweimal mit Wasser gewaschen, über Magnesiumsulfat getrocknet und vom Lösungsmittel befreit. Das zurückbleibende, farblose Oel (870 mg) wurde an Kieselgel mit Essigester/Petroläther chromatographisch gereinigt, wobei 693 mg (83 %) p-(3-Hydroxypropyl)phenyl-p-tosylat als milchiges Oel isoliert wurden.

b) Eine Lösung von 12 ml Oxalylchlorid in 380 ml Methylenchlorid wurde bei —60 °C tropfenweise mit einer Lösung von 19,8 ml Dimethylsulfoxid in 30 ml Methylenchlorid versetzt und noch 5 Minuten bei —60 °C gerührt. Anschliessend wurde das Gemisch tropfenweise mit einer Lösung von 38,9 g p-(3-Hydroxypropyl)phenyl-p-tosylat in 100 ml Methylenchlorid versetzt, noch 20 Minuten bei — 60 °C gerührt und dann mit 88,5 ml Triäthylamin versetzt. Das Reaktionsgemisch wurde noch 5 Minuten bei — 60 °C gerührt, dann langsam auf Raumtemperatur erwärmen gelassen und noch 5 Minuten bei 24 °C gerührt. Anschliessend wurde das Reaktionsgemisch mit Wasser versetzt. Die organische Phase wurde mit Wasser und Kochsalzlösung gewaschen (Nachextrahieren mit Methylenchlorid), über Magnesiumsulfat getrocknet und eingedampft. Chromatographische Trennung des zurückbleibenden, braungelben Oeles (38 g) an Kieselgel mit Essigester/Petroläther (Vol. 40 : 60) ergab 33,8 g 3-[p-(p-Tosyloxy)phenyl]propionaldehyd als hellgelbes Oel.

c) Eine Suspension von 14,87 g Methoxymethyl-triphenylphosphoniumchlorid in 130 ml t-Butylmethyläther wurde bei — 20 °C mit 5,2 g Kalium-t-butylat versetzt und ohne Kühlung noch 1,2 Stunden gerührt. Anschliessend wurde das Gemisch bei — 5 °C tropfenweise mit einer Lösung von 8,8 g 3-[p-(p-Tosyloxy)phenyl]propionaldehyd in 30 ml Tetrahydrofuran versetzt. Das Kühlbad wurde entfernt und das Gemisch noch 10 Minuten bei Raumtemperatur gerührt. Dann wurde das Reaktionsgemisch mit Wasser versetzt und zweimal in Wasser/Diäthyläther verteilt. Die wässrigen Phasen wurden mit Diäthyläther nachextrahiert. Die organischen Phasen wurden über Magnesiumsulfat getrocknet und eingedampft. Der ölige Rückstand wurde in Essigester gelöst und die Lösung mit Petroläther versetzt und durch Filtration vom ausgefallenen Triphenylphosphinoxid befreit. Nach Eindampfen des Filtrates und chromatographischer Trennung des Rückstandes an Kieselgel wurden 4,4 g (44 %) p-(4-Methoxy-3-butenyl)phenyl-p-tosylat als leicht gelbliches Oel erhalten.

d) Ein Gemisch von 4,3 g p-(4-Methoxy-3-butenyl)phenyl-p-tosylat, 60 ml Eisessig und 30 ml Wasser wurde 40 Minuten bei 110 °C gerührt, dann auf Raumtemperatur abgekühlt und mit 200 ml Wasser verdünnt. Die wässrige Phase wurde viermal mit Methylenchlorid extrahiert. Die organischen Phasen wurden zweimal mit verdünnter Natriumcarbonat-Lösung gewaschen, über Magnesiumsulfat getrocknet und vom Lösungsmittel befreit. Es resultierten 4,35 g 4-[p-(p-Tosyloxy)-phenyl]butyraldehyd als gelbliches Oel.

e) Eine Suspension von 7,32 g Methyltriphenylphosphoniumbromid in 70 ml t-Butylmethyläther wurde bei — 5 °C mit 2,45 g Kalium-t-butylat versetzt und noch 40 Minuten bei Raumtemperatur gerührt. Anschliessend wurde das Gemisch bei 0 °C langsam mit einer Lösung von 4,35 g 4-[p-(p-Tosyloxy)phenyl]butyraldehyd in 30 ml t-Butylmethyläther versetzt. Das Reaktionsgemisch wurde noch 5 Minuten bei 0 °C und 30 Minuten bei Raumtemperatur gerührt, dann mit Wasser versetzt und zweimal in Wasser/Diäthyläther verteilt. Die wässrigen Phasen wurden mit Diäthyläther nachextrahiert. Die organischen Phasen wurden über Magnesiumsulfat getrocknet und eingeengt. Der Rückstand wurde in Essigester gelöst und die Lösung mit Petroläther versetzt und durch Filtration vom ausgefallenen Triphenylphosphinoxid befreit. Nach Einengen des Filtrates und chromatographischer Trennung des zurückbleibenden, gelbes Oeles (4,3 g) an Kieselgel wurden 3,37 g (78 %) p-(4-Pentenyl)phenyl-p-tosylat als farbloses Oel erhalten.

f) Ein Gemisch von 3,35 g p-(4-Pentenyl)phenyl-p-tosylat und 50 ml 10 %-iger, äthanolischer Kaliumhydroxid-Lösung wurde 1 Stunde bei 100 °C gerührt. Anschliessend wurde das Reaktionsgemisch auf Raumtemperatur abgekühlt und mit verdünnter Salzsäure sauer gestellt. Die wässrige Phase wurde viermal mit Diäthyläther extrahiert. Die organischen Phasen wurden mit Wasser gewaschen, über Magnesiumsulfat getrocknet und eingeengt. Das zurückbleibende braune Oel (1,92 g) wurde an Kieselgel mit Essigester/Petroläther (Vol. 8 : 92) chromatographisch gereinigt, wobei 1,70 g (99 %) p-(4-Pentenyl)phenol als leicht gelbes Oel erhalten wurden.

## Beispiel 11

Ein Gemisch von 520 mg trans-4-(4-Pentenyl)cyclohexancarbonsäure, 439,4 mg p-Aethoxyphenol, 765,5 mg Dicyclohexylcarbodiimid und 45 mg 4-(Dimethylamino)pyridin wurden in 80 ml Methylenchlorid gelöst und bei Raumtemperatur 20 Stunden gerührt. Anschliessend wurde das Reaktionsgemisch mit Diäthyläther verdünnt, der ausgefallene Harnstoff abfiltriert und das Filtrat eingeengt. Der Rückstand wurde in Hexan aufgenommen und mit verdünnter Salzsäure, Natriumhydrogencarbonat-Lösung und Wasser gewaschen. Die wässrigen Phasen wurden zweimal mit Hexan nachextrahiert. Die organischen Phasen wurden über Magnesiumsulfat getrocknet und eingedampft. Niederdruckchromatographie des erhaltenen Rückstandes an Kieselgel mit Essigester/Petroläther (Vol. 3 : 97) ergab 0,7 g (83 %) trans-4-(4-Pentenyl)cyclohexancarbonsäure-p-äthoxyphenylester. Nach Umkristallisation aus 3 ml Methanol wurden schliesslich 455 mg Produkt in Form farbloser Kristalle erhalten ; Smp. (C-N) 32,7 °C, Klp. (N-I) 57,5 °C.

In analoger Weise wurden folgende Verbindungen hergestellt :

trans-4-(4-Pentenyl)cyclohexancarbonsäure-p-propyloxyphenylester ; Smp. (C-N) 32,5 °C, Klp. (N-I) 43,5 °C,

trans-4-(4-Pentenyl)cyclohexancarbonsäure-4-cyano-3-fluorphenylester ; Smp. (C-I) 21,9 °C, Klp. (N-I) — 26,9 °C,

trans-4-Pentylcyclohexancarbonsäure-p-(4-pentenyl)phenylester; Smp. (C-I) 33,3 °C, Klp. (N-I) 21,7 °C,
trans-4-(4-Pentenyl)cyclohexancarbonsäure-trans-4-butylcyclohexylester,
p-Pentylbenzoesäure-p'-(4-pentenyl)phenylester; Smp. (C-N) 5,5 °C, Klp. (N-I) — 7,6 °C,
p-(4-Pentenyl)-benzoesäure-p'-pentylphenylester; Smp. (C-N) 9,2 °C, Klp. (N-I) 14,0 °C,
p-(4-Pentenyl)benzoesäure-p'-cyanophenylester; Smp. (C-I) 38,4 °C,
p-(4-Pentenyl)benzoesäure-4-cyano-3-fluorphenylester; nicht kristallisierbar bis — 50 °C,
p-[trans-4-(4-Pentenyl)cyclohexyl]benzoesäure-p'-propylphenylester; Smp. (C-S) 73,1 °C, S-S 88 °C, S-N 109,5 °C, Klp. (N-I) 155 °C.

Beispiel 12

a) In einer Lösung von 49 g Malonsäurediäthylester in 175 ml Aethanol wurden 7,05 g Natrium aufgelöst. Die noch warme Lösung (50 °C) wurde innert 15 Minuten tropfenweise mit 45,6 g 5-Brom-1-penten versetzt und 2 Stunden zum Sieden erhitzt. Nach dem Erkalten wurde das Reaktionsgemisch auf 500 ml Diäthyläther und 300 ml halbgesättigte Kochsalzlösung gegossen. Die wässrige Phase wurde abgetrennt und zweimal mit je 200 ml Diäthyläther nachextrahiert. Die organischen Phasen wurden zweimal mit je 150 ml halbgesättigter Kochsalzlösung gewaschen, über Natriumsulfat getrocknet, filtriert und eingeengt. Chromatographische Trennung der erhaltenen, gelben Flüssigkeit (55,2 g) an Kieselgel mit Hexan/Essigester (Vol. 95 : 5) ergab 33,2 g (4-Pentenyl)malonsäurediäthylester als farblose Flüssigkeit.

b) Eine Suspension von 13,8 g Lithiumaluminiumhydrid in 500 ml Tetrahydrofuran wurde unter Stickstoff bei 0-5 °C innert 1 Stunde tropfenweise mit einer Lösung von 33,2 g (4-Pentenyl)malonsäurediäthylester in 125 ml Tetrahydrofuran versetzt. Das Gemisch wurde über Nacht bei Raumtemperatur gerührt und dann 3 Stunden zum Sieden erhitzt. Nach dem Erkalten wurde vorsichtig zuerst 25 ml Aceton und dann 25 ml gesättigte Natriumhydrogencarbonat-Lösung zum Reaktionsgemisch zugetropft. Der entstandene Brei wurde genutscht und der Rückstand auf der Nutsche viermal mit Tetrahydrofuran gewaschen. Einengen des Filtrates ergab 17,8 g 2-(4-Pentenyl)-1,3-propandiol (Reinheit 89 %) als gelbe Flüssigkeit.

c) Ein Gemisch von 3,6 g 2-(4-Pentenyl)-1,3-propandiol, 4,4 g p-Butyloxybenzaldehyd, 75 ml Toluol und 3 Tropfen 10 %-iger Schwefelsäure wurde 2,5 Stunden zum Sieden erhitzt, wobei ca. 50 ml feuchtes Lösungsmittel abdestilliert und dieses durch Zutropfen von 50 ml frischem Toluol ersetzt wurde. Danach wurde das Gemisch mit 4 Tropfen Triäthylamin neutralisiert und nach dem Erkalten zweimal mit je 50 ml 5 %-iger Natriumhydrogencarbonat-Lösung und zweimal mit je 50 ml Wasser gewaschen. Die organische Phase wurde über Natriumsulfat getrocknet und eingeengt. Der Rückstand (7,5 g) wurde an Kieselgel mit Hexan/Essigester (Vol. 92 : 8) chromatographiert, wobei 4,2 g trans-4-(p-Butyloxyphenyl)-5-(4-pentenyl)-m-dioxan erhalten wurden. Zweimalige Umkristallisation aus Hexan ergab 2,2 g reines Produkt mit Smp. (C-N) 29,5 °C und Klp. (N-I) 30,7 °C.

In analoger Weise wurden folgende Verbindungen hergestellt:
trans-2-(p-Aethoxyphenyl)-5-(4-pentenyl)-m-dioxan; Smp. (C-I) 35,6 °C, Klp. (N-I) 22,8 °C,
trans-2-(p-Propyloxyphenyl)-5-(4-pentenyl)-m-dioxan; Smp. (C-I) 36,2 °C, Klp. (S-I) 15,2 °C,
p-[trans-5-(4-Pentenyl)-m-dioxan-2-yl]benzonitril; Smp. (C-I) 38,0 °C, Klp. (N-I) 7,1 °C.

Beispiel 13

a) Eine Suspension von 91,0 g Pyridiniumchlorochromat in 650 ml Methylenchlorid wurde unter Rühren bei Raumtemperatur innert 5 Minuten tropfenweise mit einer Lösung von 22,6 g 5-Hexen-1-ol in 70 ml Diäthyläther versetzt und noch 2 Stunden gerührt. Anschliessend wurde das Gemisch mit 400 ml Diäthyläther versetzt und noch 15 Minuten gerührt. Danach wurde die Reaktionslösung vom ausgeschiedenen schwarzen Harz abdekantiert und filtriert. Fraktionierte Destillation des Filtrates unter Normaldruck ergab bei 110-122 °C 11,88 g 5-Hexenal.

b) Eine Suspension von 62,2 g Methoxymethyl-triphenylphosphoniumchlorid in 250 ml Diäthyläther wurde bei 0 °C unter Stickstoff mit 21,4 g Kalium-t-butylat versetzt. Die erhaltene orangerote Suspension wurde bei 5-10 °C innert 15 Minuten tropfenweise mit einer Lösung von 11,88 g 5-Hexenal in 65 ml Diäthyläther versetzt und noch 3 Stunden bei Raumtemperatur gerührt. Danach wurde das Reaktionsgemisch mit 7,5 g Natriumhydrogencarbonat und 125 ml Wasser versetzt und 10 Minuten gerührt. Die wässrige Phase wurde abgetrennt und mit 30 ml Diäthyläther nachextrahiert. Die organischen Phasen wurden zweimal mit je 30 ml Wasser gewaschen, über Natriumsulfat getrocknet und filtriert. Das Filtrat wurde bei 60 °C Badtemperatur unter Normaldruck vom Lösungsmittel befreit. Der Destillationsrückstand (gelbe Flüssigkeit) wurde mit 400 ml Pentan geschüttelt, bis der ungelöste Rückstand fest geworden war. Die erhaltene Suspension wurde auf — 25 °C gekühlt und filtriert. Fraktionierte Destillation des Filtrates unter Normaldruck ergab bei 120-143 °C 10,4 g 1-Methoxy-1,6-heptadien.

c) 13,2 ml Orthoameisensäuretrimethylester wurden bei 3 °C unter Inertgasatmosphäre mit 0,22 ml Bortrifluorid-diäthylätherat versetzt. Dann wurde das Gemisch unter Rühren innert 5 Minuten tropfenweise mit 2,52 g 1-Methoxy-1,6-heptadien versetzt. Das Reaktionsgemisch wurde noch 3 Stunden im Eisbad stehengelassen, dann mit 0,22 ml Triäthanolamin versetzt und im Rotationsverdampfer bei 50 °C eingeengt. Der Rückstand wurde in 30 ml Hexan gelöst und die Lösung mit 5 ml gesättigter Natriumhydrogencarbonat-Lösung und viermal mit je 5 ml Wasser gewaschen, über Natriumsulfat getrocknet und eingeengt. Kugelrohrdestillation des Rückstandes (4,0 g) bei 220 °C/9 Torr ergab 3,74 g (4-Pentenyl)malonaldehydtetramethylacetal als farblose Flüssigkeit.

d) Ein Gemisch von 3,74 g (4-Pentenyl)malonaldehydtetramethylacetal, 0,27 ml Wasser und 75 mg p-Toluolsulfonsäure-Monohydrat wurde 2 Stunden bei einer Badtemperatur von 110 °C erhitzt. Danach wurde das Gemisch mit 0,12 ml Triäthylamin versetzt, abkühlen gelassen und auf 80 ml Hexan ausgegossen. Das Reaktionsgemisch wurde zweimal mit je 15 ml gesättigter Natriumhydrogencarbonat-Lösung gewaschen, über Natriumsulfat getrocknet und filtriert. Einengen des Filtrates ergab 2,4 g rohes 3-Methoxy-2-(4-pentenyl)acrolein.

e) Eine Lösung von 2,4 g 3-Methoxy-2-(4-pentenyl)acrolein und 3,4 g p-Pentylbenzamidin-hydrochlorid in 30 ml Methanol wurde unter Rühren tropfenweise mit einer aus 0,55 g Natrium und 5 ml Methanol hergestellten Natriummethylat-Lösung versetzt. Das Reaktionsgemisch wurde über Nacht bei 50 °C gerührt und dann mit 3N Salzsäure neutralisiert (pH 5). Das Lösungsmittel wurde abdestilliert und der Rückstand in Diäthyläther und Wasser aufgenommen. Die wässrige Phase wurde mit Diäthyläther nachextrahiert. Die organische Phase wurde mit Wasser gewaschen, über Natriumsulfat getrocknet und eingeengt. Chromatographische Trennung des Rückstandes an Kieselgel mit Hexan/Essigester ergab 5-(4-Pentenyl)-2-(p-pentylphenyl)pyrimidin als farblose Flüssigkeit; Smp. (C-I) 8,0 °C, Klp. (N-I) —4,5 °C.

In analoger Weise kann folgende Verbindung hergestellt werden:

5-(4-Pentenyl)-2-(p-äthoxyphenyl)pyrimidin.

## Patentansprüche

1. Flüssigkristallines Gemisch enthaltend ein flüssigkristallines Trägermaterial und eine oder mehrere Verbindungen der allgemeinen Formel

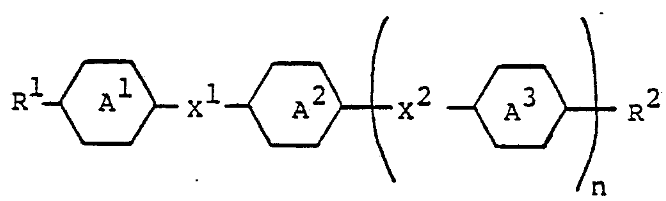

I

worin n für die Zahl 0 oder 1 steht; $X^1$ und $X^2$ einfache Kovalenzbindungen bezeichnen oder eines der Symbole $X^1$ und $X^2$ auch —COO—, —OOC— oder $—CH_2CH_2—$ bezeichnet; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $R^1$ 4-Alkenyl oder an einem Cyclohexylring auch 2Z-Alkenyl bedeutet; und $R^2$ Alkyl, Alkoxy, —CN oder —NCS bezeichnet.

2. Verwendung der Verbindungen der in Anspruch 1 definierten Formel I für elektro-optische Zwecke.

3. Verbindungen der allgemeinen Formel

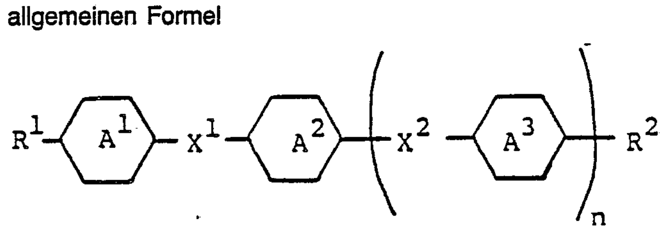

I'

worin n für die Zahl 0 oder 1 steht; $X^1$ und $X^2$ einfache Kovalenzbindungen bezeichnen oder eines der Symbole $X^1$ und $X^2$ auch —COO—, —OOC— oder $—CH_2CH_2—$ bezeichnet; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $R^1$ 4-Alkenyl oder an einem Cyclohexylring auch 2Z-Alkenyl bedeutet; und $R^2$ Alkyl, Alkoxy, —CN oder

—NCS bezeichnet; mit der Massgabe, dass nicht zugleich $R^1$ 4-Pentenyl, Ring $A^1$ trans-1,4-Cyclohexylen, $X^1$ —$CH_2CH_2$—, Ring $A^2$ 1,4-Phenylen, n die Zahl 0 und $R^2$ —CN bedeuten.

4. Verbindungen nach Anspruch 3, worin die Ringe $A^1$, $A^2$ und $A^3$ 1,4-Phenylen oder trans-1,4-Cyclohexylen darstellen oder einer dieser Ringe auch einen 2,5-disubstituierten Pyrimidinring oder einen trans-2,5-disubstituierten m-Dioxanring darstellt.

5. Verbindungen nach Anspruch 3 oder 4, worin $X^1$ und $X^2$ einfache Kovalenzbindungen bedeuten oder eines der Symbole $X^1$ und $X^2$ auch —COO— oder —OOC— bedeutet.

6. Verbindungen nach einem der Ansprüche 3-5, worin $R^1$ für einen geradkettigen Rest mit höchstens 12 Kohlenstoffatomen steht.

7. Verbindungen nach Anspruch 6, worin $R^1$ geradkettiges 4-Alkenyl mit 5 bis 12 Kohlenstoffatomen, vorzugsweise 4-Pentenyl, 4Z-Hexenyl oder 4Z-Heptenyl, bedeutet.

8. Verbindungen nach einem der Ansprüche 3 bis 7, worin $R^2$ geradkettiges $C_1$-$C_{12}$-Alkyl oder an einem Benzol- oder Pyrimidinring auch —CN, —NCS oder geradkettiges $C_1$-$C_{12}$-Alkoxy bedeutet.

9. Verfahren zur Herstellung der Verbindungen der in Anspruch 3 definierten Formel I, dadurch gekennzeichnet, dass man

a) zur Herstellung der Verbindungen der Formel I, worin $X^1$ oder $X^2$ —COO— oder —OOC— bezeichnet, eine Verbindung der allgemeinen Formel

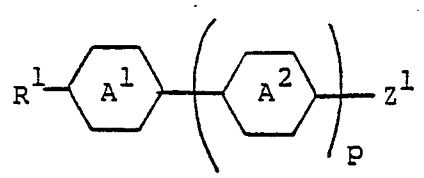

II

oder ein reaktionsfähiges Derivat hiervon mit einer Verbindung der allgemeinen Formel

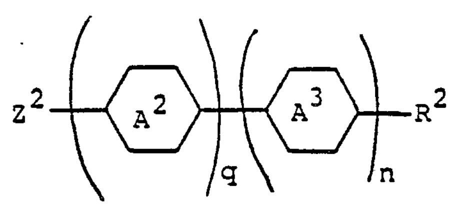

III

worin eine der Gruppen $Z^1$ und $Z^2$ —COOH und die andere —OH darstellt; einer der Indices p und q die Zahl 0 und der andere die Zahl 1 bezeichnet; und $R^1$, $R^2$, n und die Ringe $A^1$, $A^2$ und $A^3$ die in Anspruch 3 gegebenen Bedeutungen haben, oder ein reaktionsfähiges Derivat hiervon verestert, oder

b) zur Herstellung der Verbindungen der Formel I, worin einer der Ringe $A^1$, $A^2$ und $A^3$ einen trans-2,5-disubstituierten m-Dioxanring darstellt und $X^1$ und $X^2$ einfache Kovalenzbindungen oder eines dieser Symbole auch —$CH_2CH_2$— bezeichnen, eine Verbindung der allgemeinen Formel

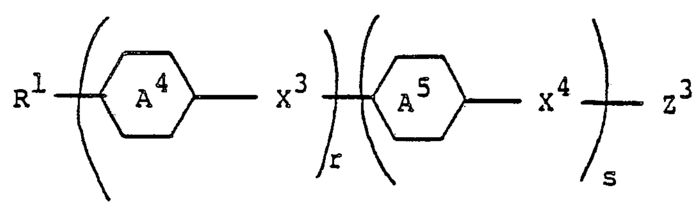

IV

mit einer Verbindung der allgemeinen Formel

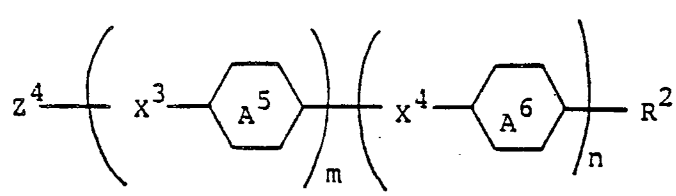

V

worin eine der Gruppen $Z^3$ und $Z^4$ —$CH(CH_2OH)_2$ und die andere —CHO darstellt; $X^3$ und $X^4$ einfache Kovalenzbindungen bezeichnen oder eines dieser Symbole auch —$CH_2CH_2$— bezeichnet; die Ringe $A^4$, $A^5$ und $A^6$ 1,4-Phenylen, 2-Fluor-1,4-phenylen oder trans-1,4-Cyclohexylen darstellen; m, n, r und s die

Zahlen 0 oder 1 bedeuten und die Summe (m + n + r + s) dieser Indices 1 oder 2 beträgt, wobei s nur dann die Zahl 1 bedeuten kann, wenn r für die Zahl 1 steht, und m nur dann die Zahl 1 bedeuten kann, wenn r für die Zahl 0 steht; und $R^1$ und $R^2$ die in Anspruch 3 gegebenen Bedeutungen haben, umsetzt, oder

c) zur Herstellung der Verbindungen der Formel I, worin einer der Ringe $A^1$, $A^2$ und $A^3$ einen 2,5-disubstituierten Pyrimidinring darstellt und $X^1$ und $X^2$ einfache Kovalenzbindungen oder eines dieser Symbole auch $—CH_2CH_2—$ bezeichnen, eine Verbindung der allgemeinen Formel IV mit einer Verbindung der Formel V, worin eine der Gruppen $Z^3$ und $Z^4$ $H_2N—\overset{|}{C}=NH \cdot HCl$ und die andere $OHC—\overset{|}{C}=CHOR^3$ darstellt, $R^3$ Alkyl bezeichnet und $X^3$, $X^4$, $R^1$, $R^2$, m, n, r, s und die Ringe $A^4$, $A^5$ und $A^6$ die obigen Bedeutungen haben, in Gegenwart einer Base umsetzt, oder

d) zur Herstellung der Verbindungen der Formel I, worin $X^1$ und $X^2$ einfache Kovalenzbindungen oder eines dieser Symbole auch $—CH_2CH_2—$ bezeichnen, eine Verbindung der allgemeinen Formel

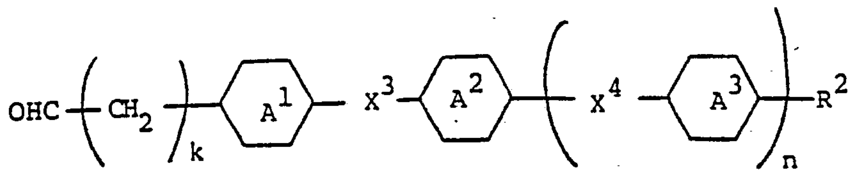

VI

worin $X^3$ und $X^4$ einfache Kovalenzbindungen bezeichnen oder eines dieser Symbole auch $—CH_2CH_2—$ bezeichnet; $R^2$, n und die Ringe $A^1$, $A^2$ und $A^3$ die in Anspruch 3 gegebenen Bedeutungen haben; und k die Zahl 3 oder, falls Ring $A^1$ trans-1,4-Cyclohexylen darstellt, auch die Zahl 1 bezeichnet, in Gegenwart einer Base mit Alkyltriphenylphosphoniumhalogenid umsetzt, oder

e) zur Herstellung der Verbindungen der Formel I, worin $R^2$ —NCS bezeichnet eine Verbindung der allgemeinen Formel

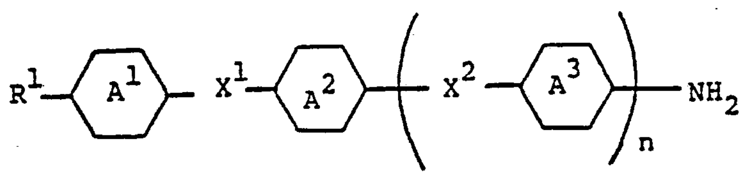

VII

worin $R^1$, $X^1$, $X^2$, n und die Ringe $A^1$, $A^2$ und $A^3$ die in Anspruch 3 gegebenen Bedeutungen haben, in Gegenwart eines Amins mit Thiophosgen umsetzt, oder

f) zur Herstellung der Verbindungen der Formel I, worin $X^1$ und $X^2$ einfache Kovalenzbindungen oder eines dieser Symbole auch $—CH_2CH_2—$ bezeichnen, die Ringe $A^1$, $A^2$ und $A^3$ 1,4-Phenylen, 2-Fluor-1,4-phenylen oder trans-1,4-Cyclohexylen darstellen und $R^2$ primäres Alkyl bedeutet, eine Verbindung der allgemeinen Formel

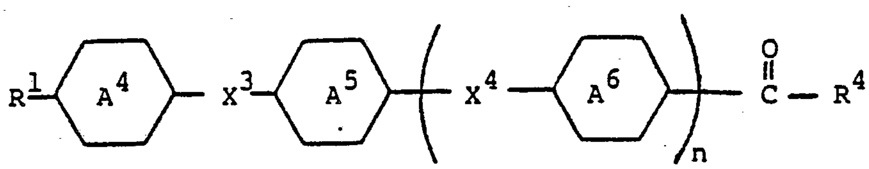

LVa

worin $X^3$ und $X^4$ einfache Kovalenzbindungen bezeichnen oder eines dieser Symbole auch $—CH_2—CH_2—$ bezeichnet; die Ringe $A^4$, $A^5$ und $A^6$ 1,4-Phenylen, 2-Fluor-1,4-phenylen oder trans-1,4-Cyclohexylen darstellen; $R^4$ Wasserstoff oder Alkyl bedeutet; und $R^1$ und n die in Anspruch 3 gegebenen Bedeutungen haben, reduziert.

10. Verbindungen der allgemeinen Formel

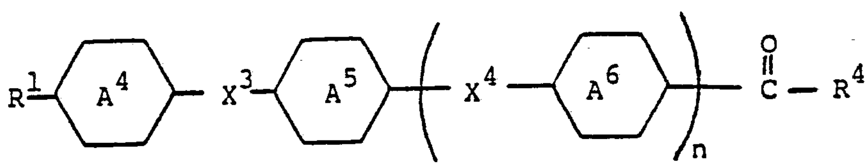

LVa

worin $X^3$ und $X^4$ einfache Kovalenzbindungen bezeichnen oder eines dieser Symbole auch $—CH_2CH_2—$ bezeichnet; die Ringe $A^4$, $A^5$ und $A^6$ 1,4-Phenylen, 2-Fluor-1,4-phenylen oder trans-1,4-Cyclohexylen darstellen; $R^4$ Wasserstoff oder Alkyl bedeutet; n für die Zahl 0 oder 1 steht; und $R^1$ 4-Alkenyl oder an einem Cyclohexylring auch 2Z-Alkenyl bedeutet.

## Claims

1. A liquid crystalline mixture containing a liquid crystalline carrier material and one or more compounds of the general formula

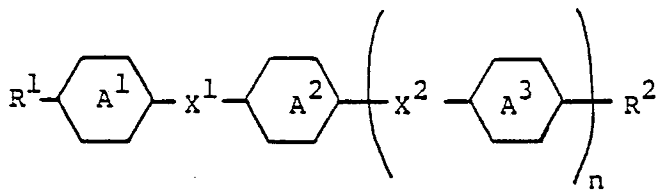

I

wherein n stands for the number 0 or 1 ; $X^1$ and $X^2$ denote single covalent bonds or one of the symbols $X^1$ and $X^2$ also denotes —COO—, —OOC— or —$CH_2CH_2$—; the rings $A^1$, $A^2$ and $A^3$ represent 1,4-phenylene, 2-fluoro-1,4-phenylene or trans-1,4-cyclohexylene or one of these rings also represents a 2,5-disubstituted pyrimidine ring or a trans-2,5-disubstituted m-dioxane ring; $R^1$ signifies 4-alkenyl or on a cyclohexyl ring also 2Z-alkenyl; and $R^2$ denotes alkyl, alkoxy, —CN or —NCS.

2. The use of the compounds of formula I defined in claim 1 for electro-optical purposes.

3. Compounds of the general formula

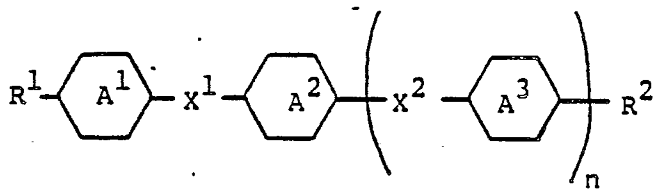

I

wherein n stands for the number 0 or 1 ; $X^1$ and $X^2$ denote single covalent bonds or one of the symbols $X^1$ and $X^2$ also denotes —COO—, —OOC— or —$CH_2CH_2$—; the rings $A^1$, $A^2$ and $A^3$ represent 1,4-phenylene, 2-fluoro-1,4-phenylene or trans-1,4-cyclohexylene or one of these rings also represents a 2,5-disubstituted pyrimidine ring or a trans-2,5-disubstituted m-dioxane ring; $R^1$ signifies 4-alkenyl or on a cyclohexyl ring also 2Z-alkenyl; and $R^2$ denotes alkyl, alkoxy, —CN or —NCS; with the proviso that simultaneously $R^1$ does not signify 4-pentenyl, ring $A^1$ does not signify trans-1,4-cyclohexylene, $X^1$ does not signify —$CH_2CH_2$—, ring $A^2$ does not signify, 1,4-phenylene, n does not signify the number 0 and $R^2$ does not signify —CN.

4. Compounds according to claim 3, wherein the rings $A^1$, $A^2$ and $A^3$ represent 1,4-phenylene or trans-1,4-cyclohexylene or one of these rings also represents a 2,5-disubstituted pyrimidine ring or a trans-2,5-disubstituted m-dioxane ring.

5. Compounds according to claim 3 or 4, wherein $X^1$ and $X^2$ signify single covalent bonds or one of the symbols $X^1$ and $X^2$ also signifies —COO— or —OOC—.

6. Compounds according to any one of claims 3-5, wherein $R^1$ stands for a straight-chain residue with a maximum of 12 carbon atoms.

7. Compounds according to claim 6, wherein $R^1$ signifies straight-chain 4-alkenyl with 5 to 12 carbon atoms, preferably 4-pentenyl, 4Z-hexenyl or 4Z-heptenyl.

8. Compounds according to any one of claims 3 to 7, whrein $R^2$ signifies straight-chain $C_1$-$C_{12}$-alkyl or on a benzene or pyrimidine ring also —CN, —NCS or straight-chain $C_1$-$C_{12}$-alkoxy.

9. A process for the manufacture of the compounds of formula I defined in claim 3, characterized by

a) for the manufacture of the compounds of formula I in which $X^1$ or $X^2$ denotes —COO— or —OOC—, esterifying a compound of the general formula

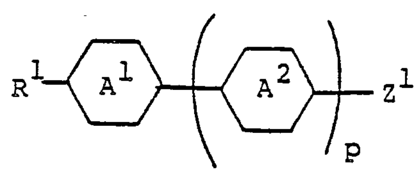

II

or a reactive derivative thereof with a compound of the general formula

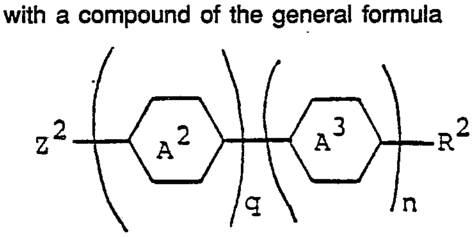

III

wherein one of the groups $Z^1$ and $Z^2$ represents —COOH and the other represents —OH ; one of the indices p and q denotes the number 0 and the other denotes the number 1 ; and $R^1$, $R^2$, n and the rings $A^1$, $A^2$ and $A^3$ have the significances given in claim 3, or a reactive derivative thereof, or

b) for the manufacture of the compounds of formula I in which one of the rings $A^1$, $A^2$ and $A^3$ represents a trans-2,5-disubstituted m-dioxane ring and $X^1$ and $X^2$ denote single covalent bonds or one of these symbols also denotes $—CH_2CH_2—$, reacting a compound of the general formula

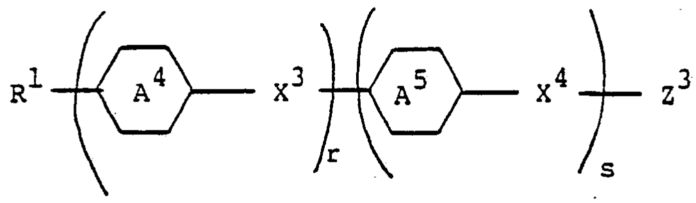

IV

with a compound of the general formula

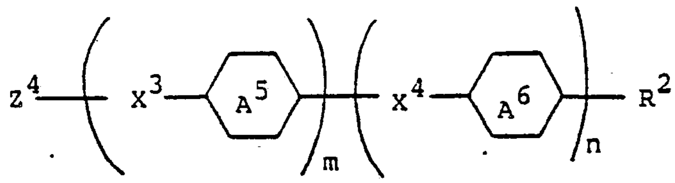

V

wherein one of the groups $Z^3$ and $Z^4$ represents $—CH(CH_2OH)_2$ and the other represents —CHO ; $X^3$ and $X^4$ denote single covalent bonds or one of these symbols also denotes $—CH_2CH_2—$ ; the rings $A^4$, $A^5$ and $A^6$ represent 1,4-phenylene, 2-fluoro-1,4-phenylne or trans-1,4-cyclohexylene ; m, n, r, and s signify the numbers 0 or 1 and the sum (m + n + r + s) of these indices is 1 or 2, whereby s can only signify the number 1 when r stands for the number 1 and m can only signify the number 1 when r stands for the number 0 ; and $R^1$ and $R^2$ have the significances given in claim 3, or

c) for the manufacture of the compounds of formula I in which one of the rings $A^1$, $A^2$ and $A^3$ represents a 2,5-disubstituted pyrimidine ring and $X^1$ and $X^2$ denote single covalent bonds or one of these symbols also denotes $—CH_2CH_2—$, reacting a compound of general formula IV with a compound of formula V in which one of the groups $Z^3$ and $Z^4$ $H_2N—\overset{|}{C}=NH \cdot HCl$ and the other represents $OHC—\overset{|}{C}=CHOR^3$, $R^3$ denotes alkyl and $X^3$, $X^4$, $R^1$, $R^2$, m, n, r, s and the rings $A^4$, $A^5$ and $A^6$ have the above significances, in the presence of a base, or

d) for the manufacture of the compounds of formula I in which $X^1$ and $X^2$ denote single covalent bonds or one of these symbols also denotes $—CH_2CH_2—$, reacting a compound of the general formula

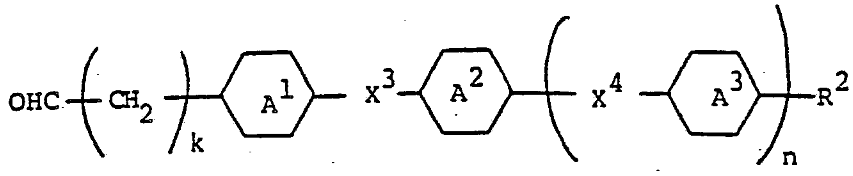

VI

wherein $X^3$ and $X^4$ denote single covalent bonds or one of these symbols also denotes $—CH_2CH_2—$ ; $R^2$, n and the rings $A^1$, $A^2$ and $A^3$ have the significances given in claim 3 ; and k denotes the number 3 or, when ring $A^1$ represents trans-1,4-cyclohexylene, also the number 1, with alkyltriphenylphosphonium halide in the presence of a base, or

e) for the manufacture of the compounds of formula I in which $R^2$ denotes —NCS, reacting a compound of the general formula

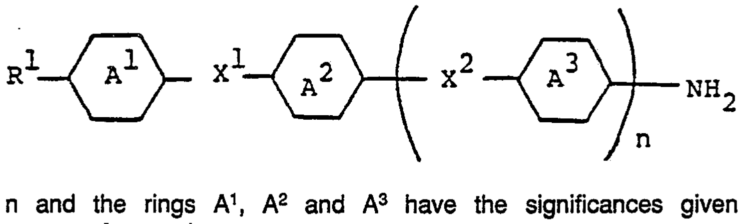

.VII

wherein $R^1$, $X^1$, $X^2$, n and the rings $A^1$, $A^2$ and $A^3$ have the significances given in claim 3, with thiophosgene in the presence of an amine, or

f) for the manufacture of the compounds of formula I in which $X^1$ and $X^2$ denote single covalent

bonds or one of these symbols also denotes $—CH_2CH_2—$, the rings $A^1$, $A^2$ and $A^3$ represent 1,4-phenylene, 2-fluoro-1,4-phenylene or trans-1,4-cyclohexylene and $R^2$ signifies primary alkyl, reducing a compound of the general formula

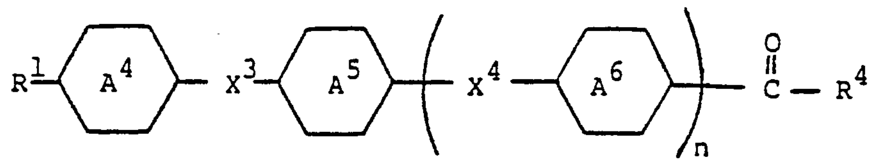

LVa

wherein $X^3$ and $X^4$ denote single covalent bonds or one of these symbols also denotes $—CH_2—CH_2—$; the rings $A^4$, $A^5$ and $A^6$ represent 1,4-phenylene, 2-fluoro-1,4-phenylene or trans-1,4-cyclohexylene; $R^4$ signifies hydrogen or alkyl; and $R^1$ and n have the significances given in claim 3.

10. Compounds of the general formula

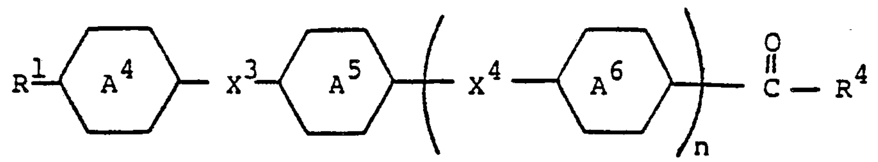

LVa

wherein $X^3$ and $X^4$ denote single covalent bonds or one of these symbols also denotes $—CH_2CH_2—$; the rings $A^4$, $A^5$ and $A^6$ represent 1,4-phenylene, 2-fluoro-1,4-phenylene or trans-1,4-cyclohexylene; $R^4$ signifies hydrogen or alkyl; n stands for the number 0 or 1; and $R^1$ signifies 4-alkenyl or on a cyclohexyl ring also 2Z-alkenyl.

## Revendications

1. Mélange à cristaux liquides contenant un véhicule à cristaux liquides et un ou plusieurs composés de formule générale

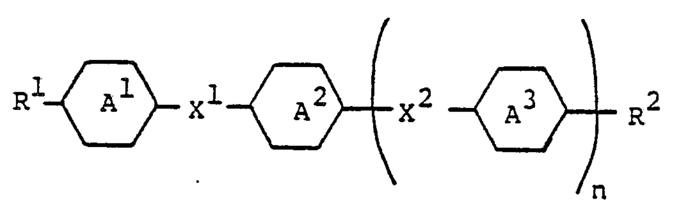

I

dans laquelle n est égal à 0 ou 1 ; $X^1$ et $X^2$ représentent des liaisons covalentes simples, l'un des symboles $X^1$ et $X^2$ pouvant également représenter —COO—, —OOC— ou $—CH_2CH_2—$ ; les cycles $A^1$, $A^2$ et $A^3$ sont des cycles 1,4-phénylène, 2-fluoro-1,4-phénylène ou trans-1,4-cyclohexylène, l'un de ces cycles pouvant également consister en un cycle pyrimidine 2,5-disubstitué ou un cycle m-dioxanne trans-2,5-disubstitué ; $R^1$ représente un groupe 4-alcényle ou bien encore, sur un noyau cyclohexyle, un groupe 2Z-alcényle ; et $R^2$ représente un groupe alkyle, alcoxy, —CN ou —NCS.

2. Utilisation des composés de formule I définie dans la revendication 1 dans des applications électrooptiques.

3. Composés de formule générale

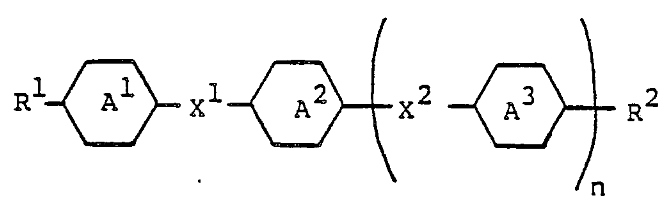

I'

dans laquelle n est égal à 0 ou 1 ; $X^1$ et $X^2$ représentent des liaisons covalentes simples, l'un de ces symboles pouvant également représenter —COO—, —OOC— ou $—CH_2CH_2—$, les cycles $A^1$, $A^2$ et $A^3$ sont des cycles 14-phénylène, 2-fluoro-1,4-phénylène ou trans-1,4-cyclohexylene, l'un de ces cycles pouvant également consister en un cycle pyrimidine 2,5-disubstitué ou un cycle m-dioxanne trans-2,5-

disubstitué ; $R^1$ représente un groupe 4-alcényle ou bien encore, sur un noyau cyclohexyle, un groupe 2Z-alcényle ; et $R^2$ représente un groupe alkyle, alcoxy, —CN ou —NCS ; étant toutefois spécifié que la combinaison de substituants suivante est exclue : $R^1$ représentant un groupe 4-pentényle, le cycle $A^1$ étant un cycle trans-1,4-cyclohexylène, $X^1$ représentant un groupe $—CH_2CH_2—$, le cycle $A^2$ étant un cycle 1,4-phénylène, n étant égal à 0 et $R^2$ représentant le groupe —CN.

4. Composés selon la revendication 3, dans lesquels les cycles $A^1$, $A^2$ et $A^3$ sont des cycles 1,4-phénylène ou trans-1,4-cyclohexylène, l'un de ces cycles pouvant également consister en un cycle pyrimidine 2,5-disubstitué ou un cycle m-dioxanne trans-2,5-disubstitué.

5. Composés selon la revendication 3 ou 4, dans lesquels $X^1$ et $X^2$ représentent des liaisons covalentes simples, l'un des symboles $X^1$ et $X^2$ pouvant également représenter —COO— ou —OOC—.

6. Composés selon l'une des revendications 3 à 5, dans lesquels $R^1$ représente un groupe à chaîne droite contenant 12 atomes de carbone au maximum.

7. Composés selon la revendication 6, dans lesquels $R^1$ représente un groupe 4-alcényle à chaîne droite en $C_5$-$C_{12}$, de préférence un groupe 4-pentényle, 4Z-hexényle ou 4Z-heptényle.

8. Composés selon l'une des revendications 3 à 7, dans lesquels $R^2$ représente un groupe alkyle à chaîne droite $C_1$-$C_{12}$ ou encore, sur un noyau benzénique ou pyrimidinique, un groupe —CN, —NCS ou un groupe alcoxy à chaîne droite en $C_1$-$C_{12}$.

9. Procédé de préparation des composés de formule I définie dans la revendication 3, caractérisé en ce que :

a) pour préparer des composés de formule I dans laquelle $X^1$ ou $X^2$ représente un groupe —COO— ou —OOC—, on estérifie un composé de formule générale

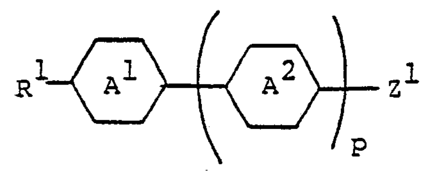

II

ou un dérivé réactif d'un tel composé, à l'aide d'un composé de formule générale

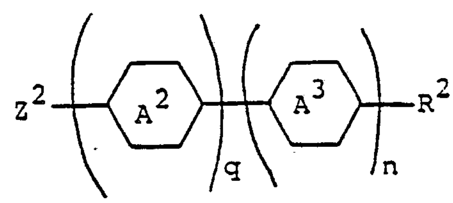

III

dans laquelle l'un des symboles $Z^1$ et $Z^2$ représente —COOH et l'autre —OH ; l'un des indices p et q est égal à 0 et l'autre à 1 ; et $R^1$, $R^2$, n et les cycles $A^1$, $A^2$ et $A^3$ sont tels que définis dans la revendication 3, ou à l'aide d'un dérivé réactif d'un tel composé, ou bien

b) pour préparer les composés de formule I dans lesquels l'un des cycles $A^1$, $A^2$ et $A^3$ est un cycle m-dioxanne trans-2,5-disubstitué et $X^1$ et $X^2$ représentent des liaisons covalentes simples, l'un de ces symboles pouvant également représenter $—CH_2CH_2—$, on fait réagir un composé de formule générale

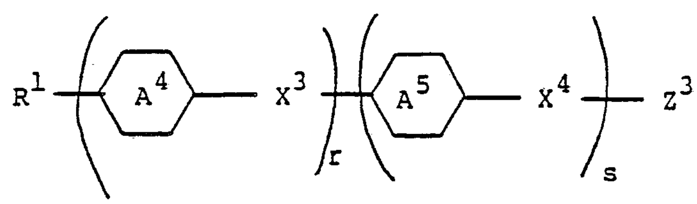

IV

avec un composé de formule générale

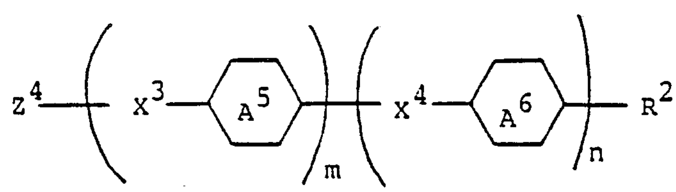

V

dans lesquelles l'un des symboles $Z^3$ et $Z^4$ représente $—CH(CH_2OH)_2$ et l'autre —CHO ; $X^3$ et $X^4$ représentent des liaisons covalentes simples, l'un de ces symboles pouvant également représenter

—$CH_2CH_2$— ; les cycles $A^4$, $A^5$ et $A^6$ sont des cycles 1,4-phénylène, 2-fluoro-1,4-phénylène ou trans-1,4-cyclohexylène ; m, n, r et s sont égaux à 0 ou 1 et la somme (m + n + r + s) de ces indices est égale à 1 ou 2, s ne pouvant être égal à 1 que si r est égal à 1, et m ne pouvant être égal à 1 que si r est égal à 0 ; et $R^1$ et $R^2$ ont les significations indiquées dans la revendication 3, ou bien

c) pour préparer les composés de formule I dans lesquels l'un des cycles $A^1$, $A^2$ et $A^3$ est un cycle pyrimidine-2,5-disubstitué et $X^1$ et $X^2$ représentent des liaisons covalentes simples, l'un de ces symboles pouvant également représenter —$CH_2CH_2$— ; on fait réagir un composé de formule générale IV avec un composé de formule V, dans lesquelles l'un des symboles $Z^3$ et $Z^4$ représente $H_2N$—C(|)=NH · HCl et l'autre OHC—C(|)=CHOR$^3$, $R^3$ représente un groupe alkyle et $X^3$, $X^4$, $R^1$, $R^2$, m, n, r, s et les cycles $A^4$, $A^5$ et $A^6$ sont tels qu'indiqué ci-dessus, en présence d'une base, ou bien

d) pour préparer les composés de formule I dans laquelle $X^1$ et $X^2$ représentent des liaisons covalentes simples, l'un de ces symboles pouvant également représenter —$CH_2CH_2$—, on fait réagir un composé de formule générale

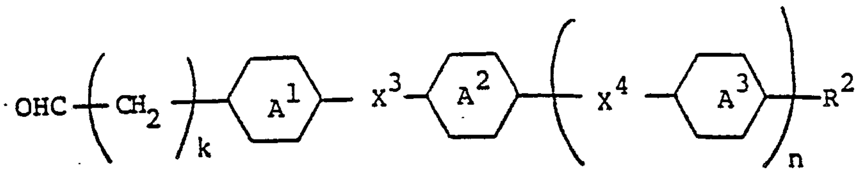

VI

dans laquelle $X^3$ et $X^4$ représentent des liaisons covalentes simples, l'un de ces symboles pouvant également représenter —$CH_2CH_2$— ; $R^2$, n et les cycles $A^1$, $A^2$ et $A^3$ sont tels qu'indiqué dans la revendication 3 ; et k est égal à 3 ou encore, lorsque le cycle $A^1$ est un cycle trans-1,4-cyclohexylène, à 1, en présence d'une base, avec un halogénure d'alkyltriphénylphosphonium, ou bien

e) pour préparer les composés de formule I dans laquelle $R^2$ représente —NCS, on fait réagir un composé de formule générale

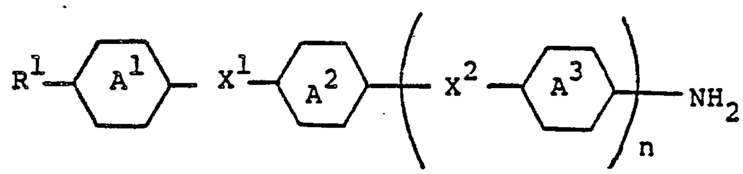

VII

dans laquelle $R^1$, $X^1$, $X^2$, n et les cycles $A^1$, $A^2$ et $A^3$ sont tels qu'indiqué dans la revendication 3, en présence d'une amine, avec le thiophosgène, ou bien

f) pour préparer les composés de formule I dans laquelle $X^1$ et $X^2$ représentent des liaisons covalentes simples, l'un de ces symboles pouvant également représenter —$CH_2CH_2$—, les cycles $A^1$, $A^2$ et $A^3$ sont des cycles 1,4-phénylène, 2-fluoro-1,4-phénylène ou trans-1,4-cyclohexylène et $R^2$ représente un groupe alkyle primaire, on réduit un composé de formule générale

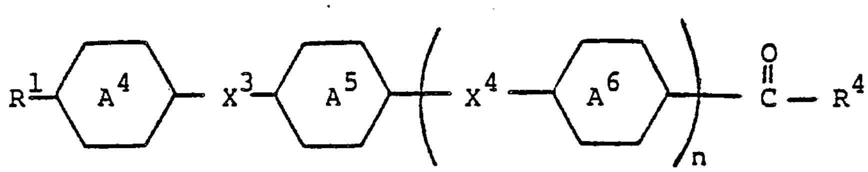

LVa

dans laquelle $X^3$ et $X^4$ représentent des liaisons covalentes simples, l'un de ces symboles pouvant également représenter —$CH_2CH_2$— ; les cycles $A^4$, $A^5$ et $A^6$ sont des cycles 1,4-phénylène, 2-fluoro-1,4-phénylène ou trans-1,4-cyclohexylène, 2-fluoro-1,4-phénylène ou trans-1,4-cyclohexylène ; $R^4$ représente l'hydrogène ou un groupe alkyle ; et $R^1$ et n ont les significations indiquées dans la revendication 3.

10. Composés de formule générale

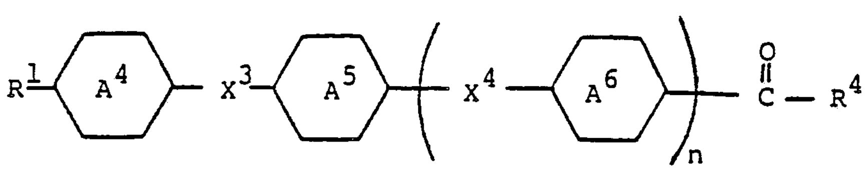

LVa

dans laquelle $X^3$ et $X^4$ représentent des liaisons covalentes simples, l'un de ces symboles pouvant également représenter $—CH_2CH_2—$ ; les cycles $A^4$, $A^5$ et $A^6$ sont des cycles 1,4-phénylène, 2-fluoro-1,4-phénylène ou trans-1,4-cyclohexylène ; $R^4$ représente l'hydrogène ou un groupe alkyle ; n est égal à 0 ou 1 ; et $R^1$ représente un groupe 4-alcényle, ou bien encore, sur un noyau cyclohexyle, un groupe 2Z-alcényle.